# EUROPEAN PATENT SPECIFICATION

(11) **EP 1 790 201 B1**
(45) Date of publication and mention of the grant of the patent: **09.02.2011**
(21) Application number: 05773639.9
(22) Date of filing: 21.07.2005
(51) Int. Cl.: H05H 1/46, H01J 37/32

(54) **PLASMA NOZZLE ARRAY FOR PROVIDING UNIFORM SCALABLE MICROWAVE PLASMA GENERATION**
PLASMADÜSENGRUPPE ZUR BEREITSTELLUNG EINER GLEICHFÖRMIGEN SKALIERBAREN MIKROWELLEN-PLASMAERZEUGUNG
RESEAU DE BUSES A PLASMA PERMETTANT LA GENERATION PAR MICRO-ONDES D'UN PLASMA ECHELONNABLE ET UNIFORME

(30) Priority: 30.07.2004 US 902435
(43) Date of publication of application: 30.05.2007
(73) Proprietor: Amarante Technologies, Inc., 3350 Scott Blvd. Building 16 Santa Clara, CA 95054 (US); Saian Corporation, Wakayama-shi Wakayama 640-8550 (JP)
(72) Inventor: LEE, Sang, Hun, San Ramon, CA 94582 (US); KIM, Jay, Joongsoo, Los Altos, CA 94024 (US)
(74) Representative: Müller-Boré & Partner Patentanwälte
(86) International application number: PCT/US2005/026280
(87) International publication number: WO 2006/014862

(56) References cited:
- EP-A- 0 547 868
- EP-A1- 0 564 082
- EP-A1- 0 578 047
- EP-A2- 0 397 468
- EP-A2- 0 420 117
- US-A- 4 185 213
- US-A- 5 173 640
- US-A1- 2002 020 691
- US-A1- 2003 000 823
- US-B1- 6 525 481
- US-B1- 6 734 385

## Description

### BACKGROUND OF THE INVENTION

### 1. Field of the Invention

The present invention relates to plasma generating systems, and more particularly to microwave plasma systems having plasma nozzle arrays.

### 2. Discussion of the Related Art

In recent years, the progress on producing plasma has been increasing. Typically, plasma consists of positive charged ions, neutral species and electrons. In general, plasmas may be subdivided into two categories: thermal equilibrium and thermal non-equilibrium plasmas. Thermal equilibrium implies that the temperature of all species including positive charged ions, neutral species, and electrons, is the same.

Plasmas may also be classified into local thermal equilibrium (LTE) and non-LTE plasmas, where this subdivision is typically related to the pressure of the plasmas. The term "local thermal equilibrium (LTE)" refers to a thermodynamic state where the temperatures of all of the plasma species are the same in the localized areas in the plasma.

A high plasma pressure induces a large number of collisions per unit time interval in the plasma, leading to sufficient energy exchange between the species comprising the plasma, and this leads to an equal temperature for the plasma species. A low plasma pressure, on the other hand, may yield one or more temperatures for the plasma species due to insufficient collisions between the species of the plasma.

In non-LTE, or simply non-thermal plasmas, the temperature of the ions and the neutral species is usually less than 100°C, while the temperature of electrons can be up to several tens of thousand degrees in Celsius. Therefore, non-LTE plasma may serve as highly reactive tools for powerful and also gentle applications without consuming a large amount of energy. This "hot coolness" allows a variety of processing possibilities and economic opportunities for various applications. Powerful applications include metal deposition systems and plasma cutters, and gentle applications include plasma surface cleaning systems and plasma displays.

One of these applications is plasma sterilization, which uses plasma to destroy microbial life, including highly resistant bacterial endospores. Sterilization is a critical step in ensuring the safety of medical and dental devices, materials, and fabrics for final use. Existing sterilization methods used in hospitals and industries include autoclaving, ethylene oxide gas (EtO), dry heat, and irradiation by gamma rays or electron beams. These technologies have a number of problems that must be dealt with and overcome and these include issues such as thermal sensitivity and destruction by heat, the formation of toxic byproducts, the high cost of operation, and the inefficiencies in the overall cycle duration. Consequently, healthcare agencies and industries have long needed a sterilizing technique that could function near room temperature and with much shorter times without inducing structural damage to a wide range of medical materials including various heat sensitive electronic components and equipment.

Atmospheric pressure plasmas for sterilization, as in the case of material processing, offer a number of distinct advantages to users. Its compact packaging makes it easily configurable, it eliminates the need for highly priced vacuum chambers and pumping systems, it can be installed in a variety of environments without additional facilitation needs, and its operating costs and maintenance requirements are minimal. In fact, the fundamental importance of atmospheric plasma sterilization lies in its ability to sterilize heat-sensitive objects, simple-to-use, and faster turnaround cycle. Atmospheric plasma sterilization may be achieved by the direct effect of reactive neutrals, including atomic oxygen and hydroxyl radicals, and plasma generated UV light, all of which can attack and inflict damage to bacteria cell membranes. Thus, there is a need for devices that can generate atmospheric pressure plasma as an effective and low-cost sterilization source.

One of the key factors that affect the efficiency of atmospheric plasma sterilization systems, as in the case of other plasma generating systems, is scalability of plasmas generated by the systems. There are several microwave nozzle based atmospheric pressure plasma systems widely used in the industrial and educational institutions around the world. The most of these designs are single nozzle based and they lack large volume scalability required for sterilization of medical devices applications. Also, such plasma systems generate high temperature plasma, which is not suitable for sterilization applications.

One solution to provide uniform plasma uses a nozzle array coupled to a microwave cavity. One of the challenging problems of such a system is controlling the microwave distribution within the microwave cavity so that the microwave energy (or, equivalently microwave) is localized at intended regions (hereinafter, referred to as "high-energy regions") that are stationary within the cavity. In such systems, plasma uniformity and scalability may be obtained by coupling nozzles to the controlled high-energy spots, which also enhances the operational efficiency of the system.

Most of the conventional systems having a microwave cavity are designed to provide a uniform microwave energy distribution in the microwave cavity. For example, Gerling, "WAVEGUIDE COMPONENTS AND CONFIGURATIONS FOR OPTIMAL PERFORMANCE IN MICROWAVE HEATING SYSTEMS," published on www.2450mhz.com by Gerling Applied Engineering Inc. in 2000, teaches a system having two rotating phase shifters. In this system, the two rotating phase shifters generate high-energy regions that continuously move within the microwave cavity to insure a uniform heating distribution within the microwave cavity.

US 5 173 640 discloses an apparatus for the production of a regular microwave field over a great area comprising a cavity resonator in which standing microwaves are generated. From the electromagnetic field energy is diverted by special coupling elements into one plasma chamber whereby a planar wave is generated within the one plasma chamber using Huygen's principle.

EP 0 547 868 A1 discloses an apparatus for controlling plasma size and position comprising a microwave chamber. Within the microwave chamber an incident wave is generated which is reflected from a sliding short movable within the microwave chamber. Both waves, i.e. the incident wave and the reflected wave, are superposed within the microwave chamber, whereby the phase shift of superposition is controlled by the sliding short. As a result the microwave energy density distribution within the microwave chamber is controllable.

US 4 185 213 discloses a plasma generator comprising a microwave cavity, whereby a plurality of tubes transparent to microwaves intersecting the microwaves cavity. A gas flows through the tubes, whereby the gas is ionised in the portion of the tubes intersecting the microwave cavity. Finally, the ionised gas is discharged through nozzles located at the end of the tubes as plasma plumes.

US 2002/0020691 A1 discloses an apparatus generating a plasma using a microwave power source and a waveguide. The waveguide having a gas entry, a gas exit and a conduit preventing gas from flowing into the interior of the waveguide. Furthermore, a grounded electrical conducting member extends from the gas entry into the waveguide to ignite the plasma within the waveguide and to sustain the plasma by coupling sufficient microwave power to the gas.

In contrast to such conventional systems, a plasma generating system that has a plasma nozzle array should be able to deterministically control the microwave in its microwave cavity and generate high-energy regions coupled to the nozzle array. Thus, there is a strong need for plasma generating systems that can deterministically generate and control high-energy regions within the microwave cavity and have plasma nozzle arrays disposed so as to receive microwave energy from the high-energy regions.

Thus, according to an aspect, it is a problem to provide a microwave plasma system for relatively cool plasma working at atmospheric pressure with an enhanced efficiency in plasma production and less production costs.

This problem is solved by a method for configuring a microwave plasma nozzle array having the features disclosed in claim 1 and a microwave plasma system having the features disclosed in claim 11. Preferred embodiments are defined in the dependent subclaims.

### SUMMARY OF THE INVENTION

According to one aspect, a method for configuring a microwave plasma nozzle array includes steps of: directing microwaves into a microwave cavity in opposing directions such that the microwaves interfere and form a standing microwave pattern that is stationary within the microwave cavity; adjusting a phase of at least one of the microwaves to control high-energy regions generated by the standing microwave pattern; and disposing a nozzle array at least partially in the microwave cavity so that one or more nozzle elements of the nozzle array are configured to receive microwave energy from a corresponding one of the high-energy regions.

According to another aspect, a method for configuring a microwave plasma nozzle array includes steps of: directing a first pair of microwaves into a microwave cavity in opposing directions along a first axis; directing a second pair of microwaves into the microwave cavity in opposing directions along a second axis, the first axis being normal to the second axis such that the first and the second pairs of microwaves interfere and form high-energy regions that are stationary within the microwave cavity, adjusting a phase of at least one of the microwaves to control the high-energy regions; and disposing a nozzle array at least partially in the microwave cavity so that one or more nozzle elements of the nozzle array are configured to receive microwave energy from a corresponding one of the high-energy regions.

According to still another aspect, a microwave plasma nozzle array unit includes: a microwave cavity; and an array of nozzles, each of the nozzles including: a gas flow tube adapted to direct a flow of gas therethrough and having an inlet portion and an outlet portion; a rod-shaped conductor axially disposed in the gas flow tube, the rod-shaped conductor having a portion disposed in the microwave cavity to receive microwaves and a tip positioned adjacent the outlet portion.

According to yet another aspect, a microwave plasma system includes: a microwave source; a pair of isolators operatively connected to the microwave source; a microwave cavity having a pair of inlets; a pair of waveguides, each of the waveguides being operatively connected to at least one of the isolators and to at least one of the inlets of the microwave cavity; a pair of non-rotating phase shifters, each of the non-rotating phase shifters being operatively connected to at least one of the waveguides and to at least one of the isolators; and an array of nozzles, each of the nozzles of the array including: a gas flow tube adapted to direct a flow of gas therethrough and having an inlet portion and an outlet portion; a rod-shaped conductor being axially disposed in the gas flow tube, the rod-shaped conductor having a portion disposed in the microwave cavity to receive microwaves and a tip positioned adjacent the outlet portion.

According to another aspect, a microwave plasma system includes: a microwave source; an isolator operatively connected to the microwave source; a microwave cavity having an inlet; a waveguide operatively connected to the isolator and to the inlet of the microwave cavity; a non-rotating phase shifter operatively connected to the waveguide and the isolator; a circulator operatively connected to the waveguide and being configured to direct microwaves to the non-rotating phase shifter; a sliding short circuit operatively connected to the microwave cavity; and an array of nozzles, each of the nozzles of the array including: a gas flow tube adapted to direct a flow of gas therethrough and having an inlet portion and an outlet portion; a rod-shaped conductor being axially disposed in the gas flow tube, the rod-shaped conductor having a portion disposed in the microwave cavity to receive microwaves and a tip positioned adjacent the outlet portion.

According to another aspect, a microwave plasma system includes: a microwave source; a pair of isolators operatively connected to the microwave source; a microwave cavity having a pair of inlets; a pair of waveguides, each of said waveguides being operatively connected to a corresponding one of said isolators and to a corresponding one of said inlets of the microwave cavity; a pair of non-rotating phase shifters, each of said non-rotating phase shifters being operatively connected to a corresponding one of said waveguides and to a corresponding one of said isolators; a pair of sliding short circuits, each of said sliding short circuits being operatively connected to said microwave cavity; and an array of nozzles, each of the nozzles of the array including: a gas flow tube adapted to direct a flow of gas therethrough and having an inlet portion and an outlet portion; a rod-shaped conductor being axially disposed in the gas flow tube, the rod-shaped conductor having a portion disposed in the microwave cavity to receive microwaves and a tip positioned adjacent the outlet portion.

According to another aspect, a microwave plasma system, comprising: a microwave source; a microwave cavity having four inlets; four waveguides, each of the waveguides being operatively connected to at least one of the inlets of the microwave cavity and the microwave source; four non-rotating phase shifters, each of the non-rotating phase shifters being operatively connected to at least one of the waveguides and the microwave source; four circulators, each of the circulators being operatively connected to at least one of the waveguides and being configured to direct microwaves generated by the microwave source to at least one of the non-rotating phase shifters; and an array of nozzles, each of the nozzles of the array including: a gas flow tube adapted to direct a flow of gas therethrough and having an inlet portion and an outlet portion; and a rod-shaped conductor being axially disposed in the gas flow tube, the rod-shaped conductor having a portion disposed in the microwave cavity to receive microwaves and a tip positioned adjacent the outlet portion.

These and other advantages and features of the invention will become apparent to those persons skilled in the art upon reading the details of the invention as more fully described below.

### BRIEF DESCRIPTION OF THE DRAWINGS

FIG. 1 is a schematic diagram of a system having a plasma nozzle array in accordance with one embodiment of the present invention.
FIG. 2A schematically illustrates the interference of two microwaves within the microwave cavity of the system shown in FIG. 1, where the microwaves travel in opposing directions.
FIG. 2B schematically shows a distribution of high-energy regions within the microwave cavity for the system shown in FIG. 1.
FIG. 3 is a schematic diagram of a system having a plasma nozzle array in accordance with another embodiment of the present invention.
FIG. 4A shows a top view of the microwave cavity and plasma nozzle array shown in FIG. 1.
FIG. 4B shows a cross-sectional view of the microwave cavity and nozzle depicted in FIG. 4A taken along the line IV-IV.
FIG. 4C shows a cross-sectional view of an alternative embodiment of the microwave cavity and nozzle array depicted in FIG. 4B.
FIG. 4D shows a cross-sectional view of another alternative embodiment of the microwave cavity and nozzle array depicted in FIG. 4B.
FIG. 5A shows a top view of an alternative embodiment of the plasma nozzle array shown in FIG. 4A.
FIG. 5B shows a cross-sectional view of the microwave cavity and nozzle array depicted in FIG. 5A taken along the line IV'-IV'.
FIG. 5C shows a cross-sectional view of an alternative embodiment of the microwave cavity and nozzle array depicted in FIG. 5B.
FIG. 5D shows a cross-sectional view of another alternative embodiment of the microwave cavity and nozzle array depicted in FIG. 5B.
FIGS. 6A-6F show cross-sectional views of alternative embodiments of the microwave plasma nozzle depicted in FIG. 4C, illustrating additional components for enhancing nozzle efficiency.
FIG. 7 is a schematic diagram of a system having a plasma nozzle array in accordance with another embodiment of the present invention.
FIG. 8 shows an interference pattern of high-energy regions found within the microwave cavity of the system shown in FIG. 7, illustrating one arrangement of the nozzle array in the high-energy regions.
FIG. 9 is a schematic diagram of a microwave cavity and waveguides for generating high-energy regions in a two-dimensional array form in accordance with still another embodiment of the present invention.
FIG. 10 shows an alternative interference pattern of high-energy regions found within the microwave cavity of the systems shown in FIGS. 7 and 9, illustrating an alternative arrangement of the nozzle array in the high-energy regions.
FIG. 11 is a schematic diagram of a system having a plasma nozzle array in accordance with yet another embodiment of the present invention.
FIG. 12 shows a cross-sectional view of the microwave cavity and the nozzle array depicted in FIG. 11 taken along a direction normal to the z-axis.
FIG. 13 is an exploded perspective view of the nozzle shown in FIG. 12.
FIGS. 14A-14I show cross-sectional views of alternative embodiments of the rod-shaped conductor depicted in FIG. 13.
FIG. 15 shows a flowchart illustrating exemplary steps for coupling a microwave nozzle array in accordance with at least one embodiment of the present invention.

### DETAILED DESCRIPTION OF THE PREFERRED EMBODIMENTS

The following detailed description is of the best currently contemplated modes of carrying out the invention. The description is not to be taken in a limiting sense, but is made merely for the purpose of illustrating the general principles of the invention, since the scope of the invention is best defined by the appended claims.

It must be noted that, as used herein and in the appended claims, the singular forms "a", "and", and "the" include plural referents unless the context clearly dictates otherwise. Thus, for example, reference to "a nozzle" includes one or more nozzles and equivalents thereof known to those skilled in the art, and so forth.

As mentioned previously, conventional microwave plasma systems generate a uniform power distribution within a microwave cavity by controlling phase differences between two microwaves transmitted to the microwave cavity. Unlike existing systems, the present invention provides methods and systems for controlling the phases of the microwaves so that they can generate stationary high-energy regions within microwave cavities. Also methods for configuring a plasma nozzle array so as to use power from the high-energy regions are disclosed.

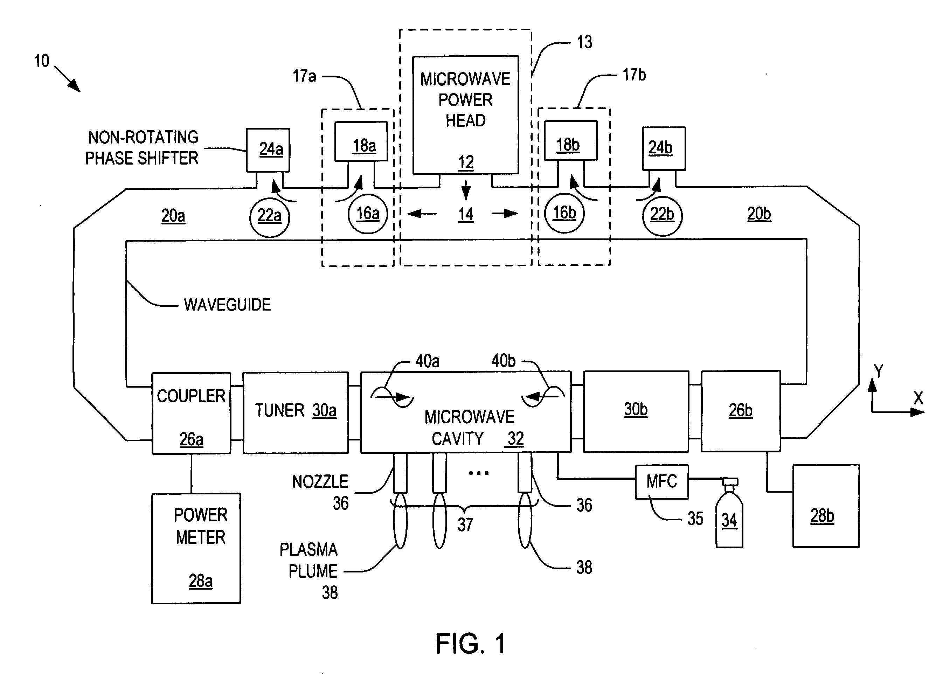

FIG. 1 is a schematic diagram of a system 10 having a plasma nozzle array in accordance with one embodiment of the present invention. As illustrated, the system 10 comprises: a microwave source 13 having a microwave power head 12 that generates microwaves and a power splitter 14 having two outlets that split the microwaves generated by the microwave power head 12; a pair of isolators 17a and 17b configured to dissipate retrogressing microwaves that travel toward the microwave power head 12, each isolator including a dummy load 18a and 18b for dissipating the retrogressing microwaves and a circulator 16 for diverting the retrogressing microwaves to the corresponding dummy load 18a and 18b; a pair of non-rotating phase shifters 24a and 24b for shifting the phases of the microwaves; a pair of circulators 22a and 22b for directing microwaves from the power splitter 14 to the non-rotating phase shifters 24a and 24b, respectively; waveguides 20a and 20b for transmitting microwaves; and a microwave cavity 32. In an alternative embodiment, the system 10 may further comprise: couplers 26a and 26b connected to power meters 28a and 28b for measuring microwave fluxes; and tuners 30a and 30b for matching impedance of microwaves. Typically, the microwave power head 12 includes a microwave generator and a power supply, which are not shown in FIG. 1 for simplicity. In another alternative embodiment, an isolator may be located between the microwave power head 12 and the two-outlet power splitter 14, thereby replacing the pair of isolators 17a and 17b.

A nozzle array 37 comprising one or more nozzles 36 is connected to the microwave cavity 32 and generate plasma plumes 38a to 38n from a gas provided from a gas tank 34 through a mass flow control (MFC) valve 35. Several embodiments of the nozzles 36 and the microwave cavity 32 that may be used for the system 10 are discussed in a copending PCT Application entitled "Microwave Plasma Nozzle with Enhanced Plume Stability and Heating Efficiency," filed on July 5, 2005, which is hereby incorporated by reference in its entirety.

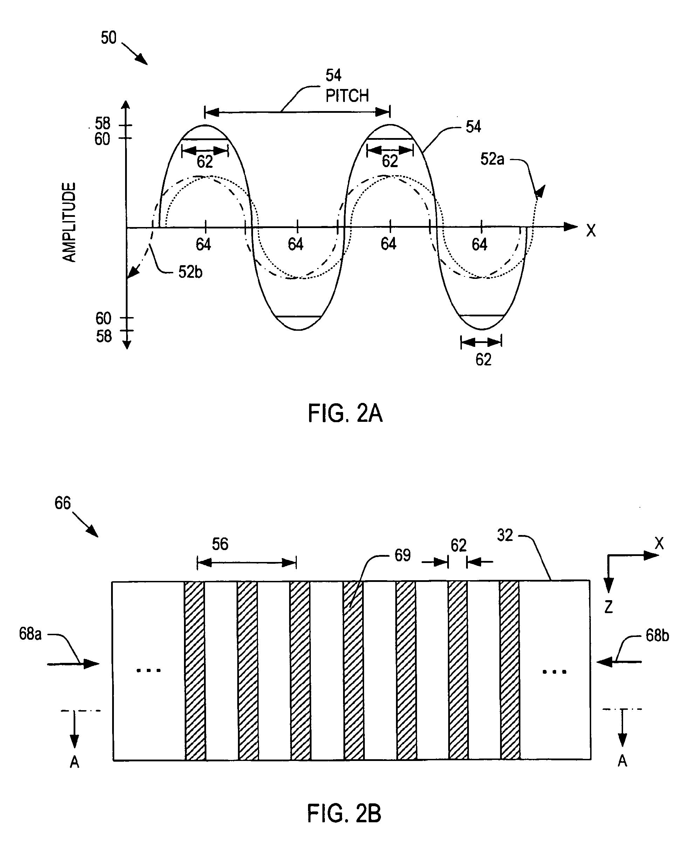

The microwaves 40a and 40b transmitted from the power splitter 14 travel in opposing directions along an x-axis within the microwave cavity 32 and yield an interference pattern, as shown in FIG. 2A. FIG. 2A shows a plot 50 of microwaves 52a and 52b that interfere with each other to yield a standing microwave 54 within the microwave cavity 32. The abscissa and ordinate of the plot 50 represent the direction of microwave propagations and amplitude of microwaves, respectively. Since the intensity of the standing microwave 54 is proportional to the square of amplitude, the standing microwave 54 has peak locations 64 for each cycle where the amplitude reaches its maximum amplitude 58. (For simplicity, hereinafter, the amplitude refers to the absolute value of the amplitude.)

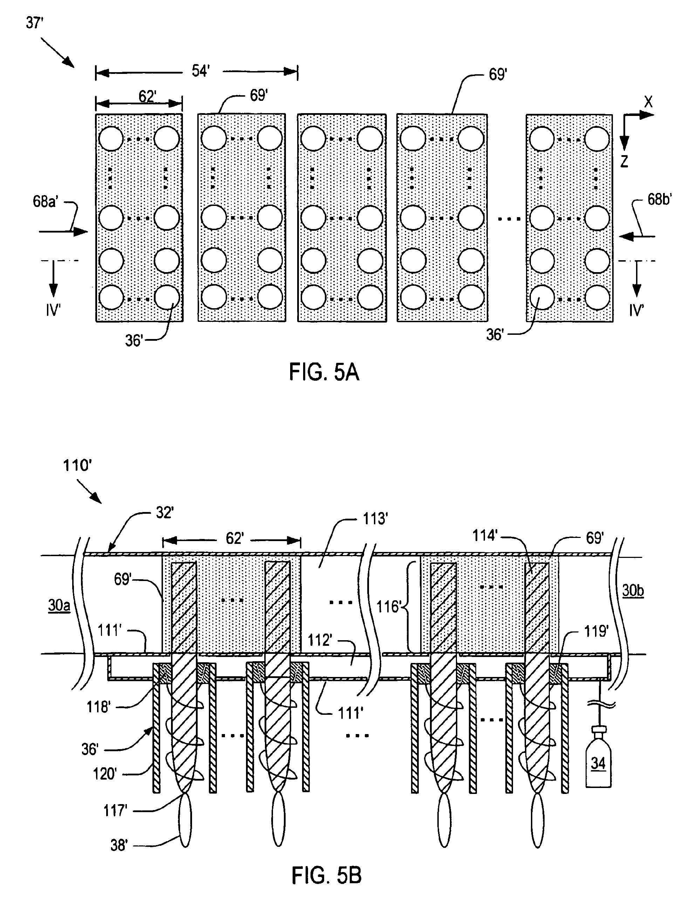

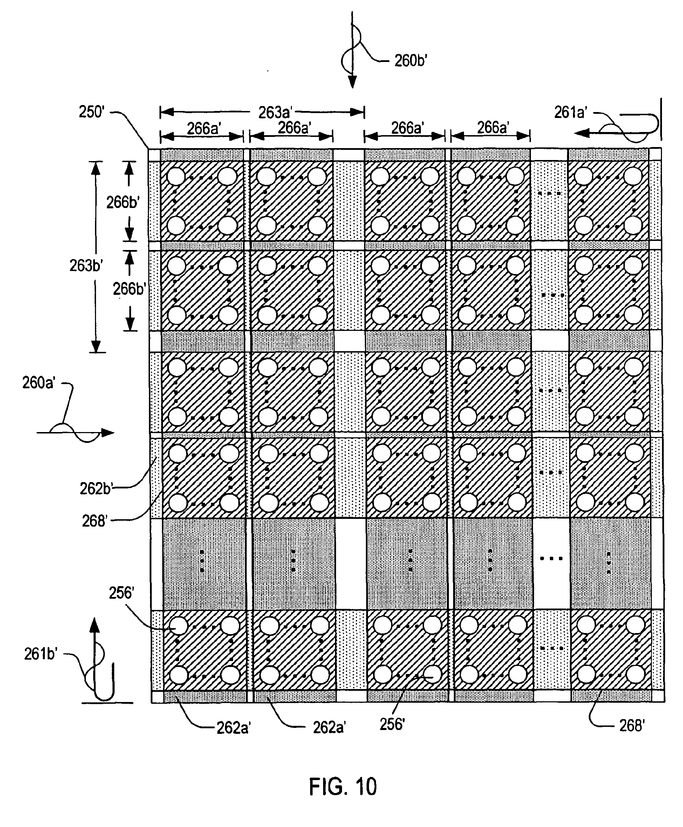

High-energy regions 69 may refer to the locations where the amplitude of the standing microwave 54 exceeds a threshold 60 that may be set by a user. As will be explained in connection with FIGS. 5A and 10, more than one nozzle may be located along x-direction in each high-energy region 69. In such cases, the width 62 of the high-energy regions 69 may be determined considering the dimension of the nozzles, spacing between two neighboring nozzles and the value of the maximum amplitude 58. For example, the user may set the threshold 60 to 75 % of the maximum amplitude 58 to provide microwave energy for the entire nozzles in the high-energy regions 69.

Peak locations 64 and maximum amplitudes 58 of the peaks as well as a width 62 of the high-energy regions 69 may be controlled by the non-rotating phase shifters 24a and 24b, while a pitch 56 is determined by the wavelength of the microwaves 52a and 52b. If the phase difference between the microwaves 52a and 52b decreases, the maximum amplitude 58 and the width 62 of the high-energy regions 69 increase. If the phases of two microwaves 52a and 52b are shifted in one direction along the x-axis, the peak locations 64 may shift in that direction.

FIG. 2B shows a distribution 66 of the high-energy regions 69 within the microwave cavity 32 viewed in a direction normal to the x-z plane. As shown in FIG. 2B, the high-energy regions 69 are generated by interference of the microwaves 52a and 52b propagating in the directions 68a and 68b, respectively, within the microwave cavity 32. As the microwaves 52a and 52b may be one-dimensional waves, each of the high-energy regions 69 may be in a rectangular strip shape and spaced by half of the pitch 56. In FIGS. 2A and 2B, the microwave cavity is assumed to be a rectangular parallelepiped for the purpose of illustration. However, it should be apparent to those of ordinary skill in the art that the microwave cavity can have any other shape without deviating from the present invention.

In an alternative embodiment, microwave source 13 may be replaced by two separate microwave power heads and two isolators attached thereto, respectively, where each microwave power head may transmit a microwave to the microwave cavity 32. In this embodiment, two microwaves 52a and 52b may have different wavelengths and amplitudes. However, by applying the same principle set forth above, the non-rotating phase shifters 24a and 24b can be used to control the peak locations 64 and the maximum amplitude 58 as well as the width 62 of high-energy regions 69.

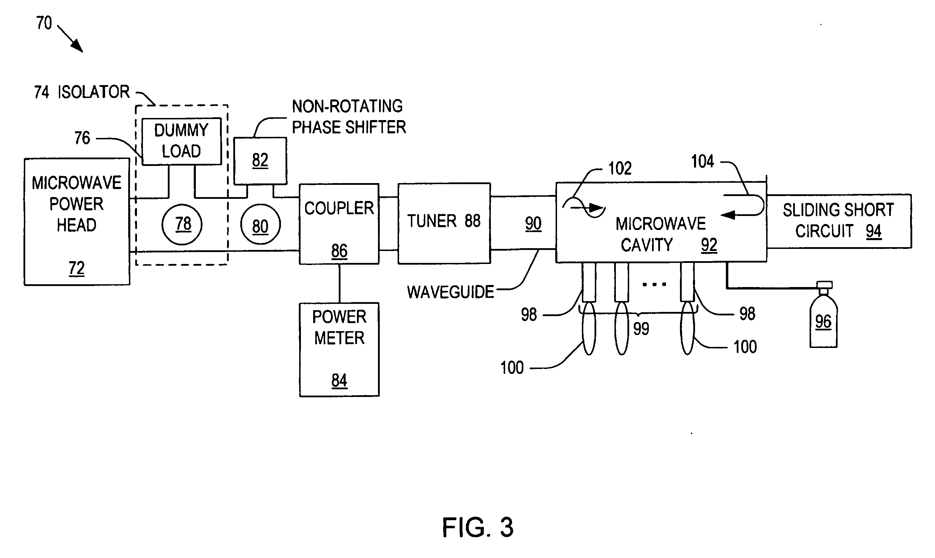

FIG. 3 is a schematic diagram of a system 70 for deterministically generating high-energy regions within a microwave cavity in accordance with another embodiment of the present invention. As illustrated, the system 70 may include a microwave power head 72 for generating microwaves; an isolator 74 having a dummy load 76 configured to dissipate the retrogressing microwaves that propagate toward the microwave power head 72 and a circulator 78 for diverting the retrogressing microwave to a dummy load 76; a non-rotating phase shifter 82 for controlling a microwave phase; a circulator 80; a microwave cavity 92; a waveguide 90 for transmitting microwaves from the microwave power head 72 to the microwave cavity 92; and a sliding short circuit 94 for controlling the phase of the reflected microwaves. In an alternative embodiment, the system 70 may further include a coupler 86 connected to power meters 84 for measuring microwave fluxes; and a tuner 88 for matching the impedance of the microwaves. In another alternative embodiment, the sliding short circuit 94 may be replaced by a wall, where the dimensions of the microwave cavity 92 along the microwave propagation is a multiple of half a wavelength of the microwaves. A nozzle array 99 comprising nozzles 98 may be connected to the microwave cavity 92 and generate plasma plumes 100 from a gas provided from a gas tank 96. The specific details of the nozzles 98 will be discussed below.

In FIG. 3, an inset diagram 102 illustrates the propagation of microwaves transmitted from the microwave power head 72 to the microwave cavity 92. The transmitted microwaves are reflected from the sliding short circuit 94, as indicated by an arrow 104, and they interfere with the incoming microwaves to generate standing microwaves within the microwave cavity 92. The sliding short circuit 94 can control the phase of the reflected microwaves and, if it is used in conjunction with a non-rotating phase shifter 82, control the locations and the maximum amplitude of the standing waves as well as the width of high-energy regions that are similar to the high-energy regions 69 shown in FIG. 2B.

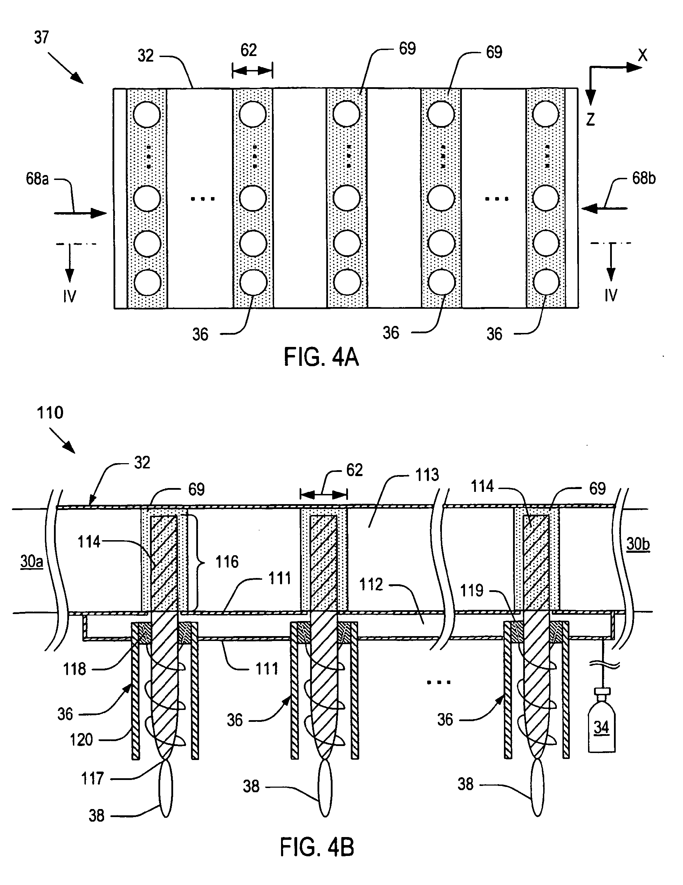

FIG. 4A is a top view of the plasma nozzle array 37 shown in FIG. 1., illustrating the nozzles 36 located within the high-energy regions 69 established within the microwave cavity 32 by microwaves traveling in opposing directions 68a and 68b. As illustrated, the nozzle array shown at 37 is described as a two-dimensional array. However, it should be apparent to those of ordinary skill that other arrangements of nozzles may be used. For example, the nozzle array 37 may have only a one-dimensional array of the nozzles 36 arranged in either the z-direction or the x-direction. It is noted that a nozzle array 99 in FIG. 3 may have the same arrangement as shown in FIG. 4A.

FIG. 4B shows a cross-sectional diagram 110 of the microwave cavity and nozzle array depicted in FIG. 4A taken along the direction IV-IV. As illustrated, the microwave cavity 32 includes a wall 111 that forms a gas flow channel 112 for admitting a gas from the gas tank 34; and a cavity 113 for receiving microwaves transmitted from the microwave source 13 and generating the high-energy regions 69. Each nozzle 36 may include a gas flow tube 120 connected to the cavity wall 111 to receive a gas through the gas flow channel 112; a rod-shaped conductor 114 having a portion 116 for collecting microwaves from the high-energy regions 69 in the cavity 113; and a vortex guide 118 disposed between the rod-shaped conductor 114 and the gas flow tube 120. The vortex guide 118 has at least one opening 119 for producing a helical swirl flow path around the rod-shaped conductor 114. The microwaves received by the rod-shaped conductor portion 116 are focused on its tapered tip 117 to generate the plasma plumes 38 using the gas. The gas flow tube 120 may be made of a material that is substantially transparent to microwaves. For example, the gas flow tube 120 may be made of a dielectric material, such as quartz.

The width 62 of the high-energy regions 69 may be optimized by controlling the non-rotating phase shifters 24a and 24b. In general, a smaller width of high-energy regions 69 may yield a higher operational efficiency of the nozzles 36. However, considering the potential variation of the high-energy regions 69 during operation of the system 10, the width 62 of the high-energy regions 69 may be slightly larger than the diameter of the rod-shaped conductor 114.

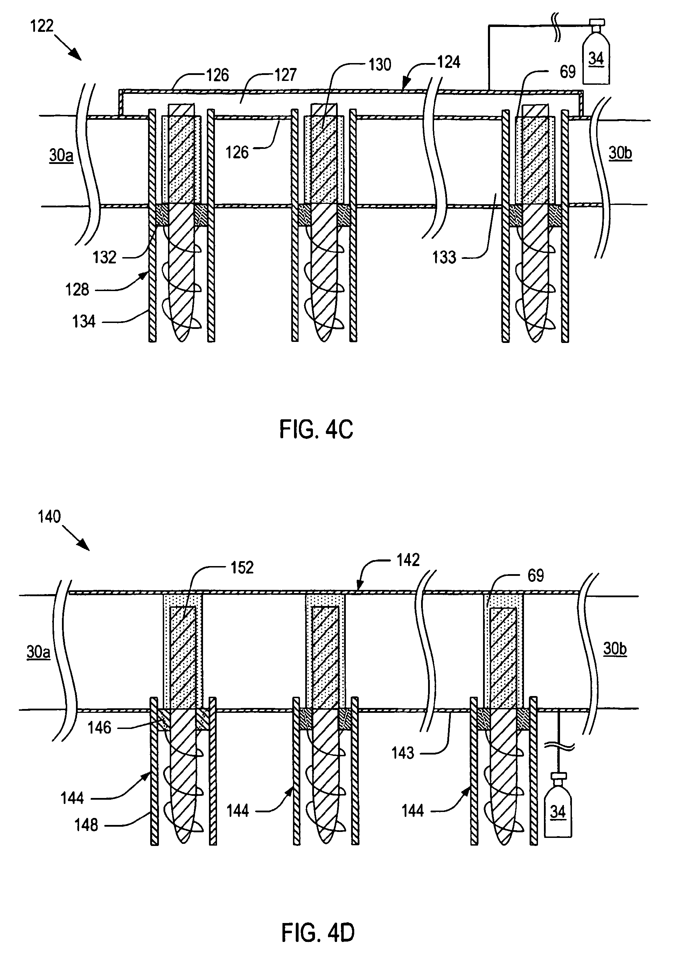

FIG. 4C is a cross-sectional diagram of an alternative embodiment 122 of the microwave cavity and nozzle array depicted in FIG. 4B. As illustrated, a nozzle 128 has similar components as those shown in FIG. 4B. FIG. 4C includes a gas flow tube 134 sealingly connected to a wall 126 to a receive a gas through a gas flow channel 127; a rod-shaped conductor 130 for collecting microwaves from the high-energy regions 69 in a cavity 133; and a vortex guide 132. The gas flow tube 134 may be made of any material that is substantially transparent to microwaves (i.e., microwaves can pass through the gas flow tube 134 with very low loss of energy) and, as a consequence, the gas flowing through the gas flow tube 134 may be pre-heated within the cavity 133 prior to reaching the region of the tapered tip of the rod-shaped conductor 130.

FIG. 4D shows a cross-sectional view of another alternative embodiment 140 of the microwave cavity and nozzle array depicted in FIG. 4A. As illustrated, nozzles 144 have components similar to their counterparts in FIG. 4B: a gas flow tube 148 sealingly connected to a wall 143 of a microwave cavity 142 to receive a gas; a rod-shaped conductor 152 for collecting microwaves from the high-energy regions 69; and a vortex guide 146. The microwave cavity 142 may form a gas flow channel connected to the gas tank 34. The rod-shaped conductor 152 may be similar to the conductor 114 illustrated in FIG. 4B where the portion 116 of the rod-shaped conductor 114 is inserted into the cavity 113 to receive microwaves. Then, the received microwaves travel along the surface thereof and are focused on the tapered tip.

A mentioned previously, the width 62 (FIG. 2) of the high-energy regions 69 may be optimized by controlling the non-rotating phase shifters 24a and 24b. In general, a smaller width of high-energy regions 69 may yield a higher operational efficiency of the nozzles 36. For this reason, in FIGS. 4A-4D, the width 62 of the high-energy regions 69 may be slightly larger than the diameter of the rod-shaped conductor 114. In these applications, the interval between two neighboring nozzles in x-direction may be half wavelength of the microwaves traveling in opposing directions 68a and 68b. However, in some applications, the interval of half-wavelength may introduce fluctuations in plasma characteristics along the x-direction and, as a consequence, a smaller interval between nozzles may be required. FIGS. 5A-5D illustrate nozzle arrays having various intervals between two neighboring nozzles in x-direction.

FIG. 5A is a top view of an alternative embodiment 37' of the plasma nozzle array shown in FIG. 4A, illustrating nozzles 36' located within high-energy regions 69' that are established by microwaves traveling in opposing directions 68a' and 68b'. As depicted, the width 62' of the high-energy region 69' may be large enough to accommodate one or more nozzles 36' in x-direction, even though the pitch 54' is equal to the wavelength of the microwaves. The width 62' may be controlled by varying the phase difference between the microwaves 68a' and 68b' as described in connection with FIG. 2A. It is noted that a nozzle array 99 in FIG. 3 may have the same arrangement as shown in FIG. 5A.

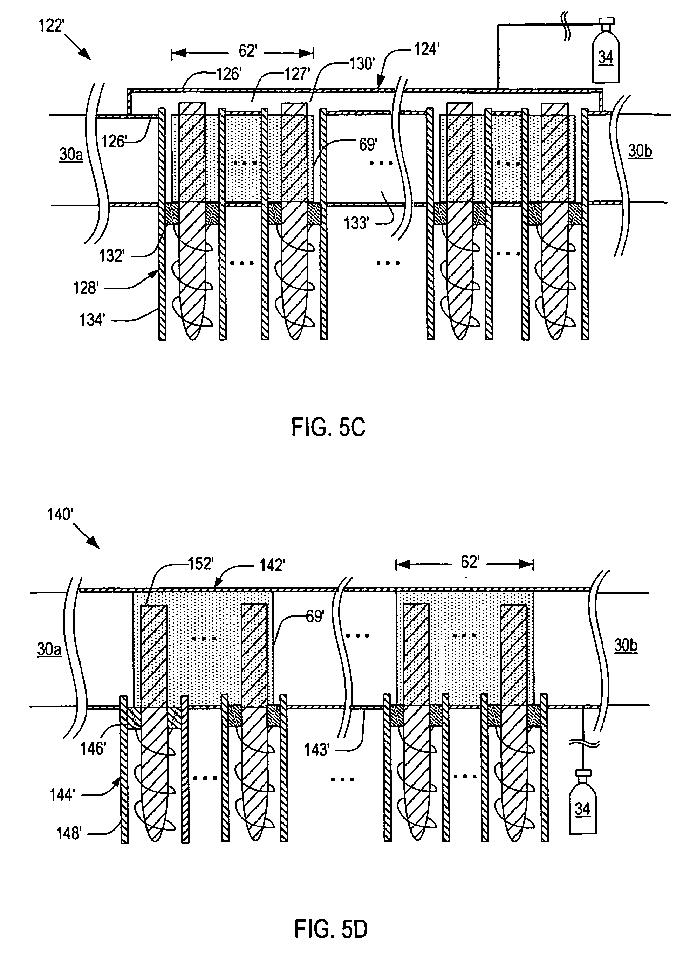

FIGS. 5B-5D are cross-sectional views of various embodiments of the microwave cavity and nozzle array in FIG. 5A taken along the line IV'-IV'. As illustrated, the three embodiments shown at 110' (FIG. 5B), 122' (FIG. 5C) and 140' (FIG. 5D) are similar to their counterparts shown at 110, 122 and 140, respectively, with the difference that the width 62' may be large enough to accommodate more than one nozzle in x-direction.

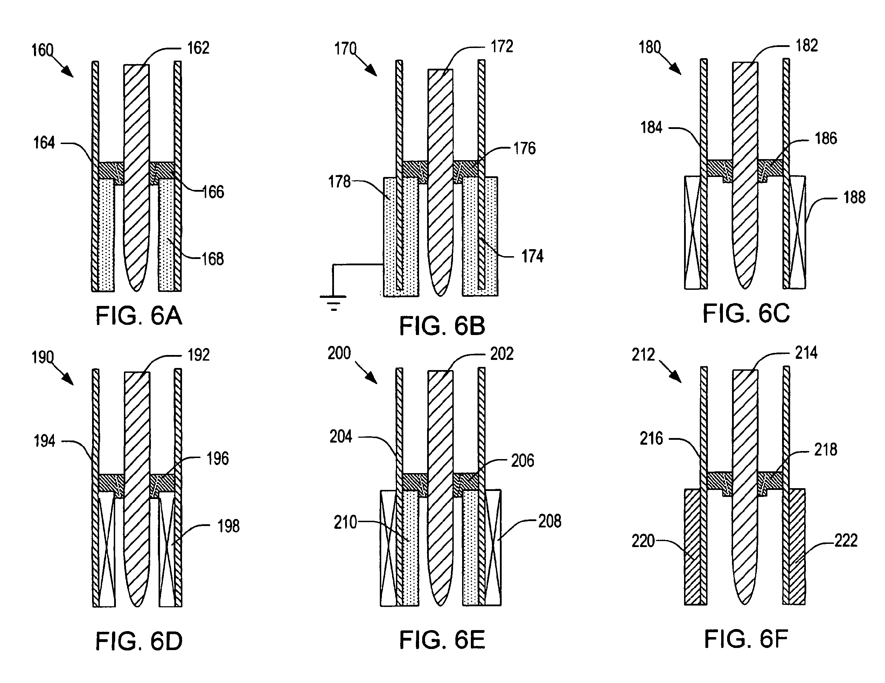

Each nozzle depicted in FIGS. 4B-4D and 5B-5D includes a rod-shaped conductor that has a portion inserted into the cavity to receive microwaves. Then, the received microwaves travel along the surface thereof and are focused on the tapered tip. Since a portion of the traveling microwaves may be lost through the gas flow tube, a shielding mechanism may be used to enhance the efficiency of the nozzles, which are illustrated in FIGS. 6A-6B.

FIG. 6A shows a cross-sectional view of a nozzle 160 which is an alternative embodiment of the nozzle 36 shown in FIG. 4C. As illustrated, the nozzle 160 includes: a rod-shaped conductor 162; a gas flow tube 164; a vortex guide 166; and an inner shield 168 for reducing microwave loss through the gas flow tube 164. The inner shield 168 has a tubular shape and engages a recess formed along an outer surface of the vortex guide 166. The inner shield 168 may provide additional control of the helical swirl around the rod-shaped conductor 162 and increase the plasma stability by changing the gap between the gas flow tube 164 and the rod-shaped conductor 162.

FIG. 6B is a cross-sectional view of another nozzle 170 which is another alternative embodiment of the nozzle 36 shown in FIG. 4C. As illustrated, the nozzle 170 includes: a rod-shaped conductor 172; a gas flow tube 174; a vortex guide 176; and a grounded shield 178 for reducing microwave power loss through the gas flow tube 174. The grounded shield 178 may cover a portion of the gas flow tube 174 that is outside of the microwave cavity. Like the inner shield 168, the grounded shield 178 may provide the additional control of the helical swirl around the rod-shaped conductor 172 and increase the plasma stability by changing the gap between the gas flow tube 174 and the rod-shaped conductor 172.

As mentioned above, the main heating mechanism applied to the nozzles shown in FIGS. 4B-4D and 5B-5D are the microwaves that are focused and discharged adjacent the tapered tip of the rod-shaped conductor, where the nozzles may produce non-LTE plasmas for sterilization. In non-LTE plasmas, the temperature of ions and neutral species may be less than 100°C, while the temperature of electrons can be up to several tens of thousand degrees in Celsius. Thus, such plasmas are highly electronically excited. To enhance the electronic temperature and increase the nozzle efficiency, the nozzles may include additional mechanisms that electronically excite the gas while the gas is within the gas flow tube, as illustrated in FIGS. 6C-6F.

FIG. 6C is a cross-sectional view of a nozzle 180 which is still another alternative embodiment of the nozzle 36 shown in FIG. 4C. As illustrated, the nozzle 180 includes: a rod-shaped conductor 182; a gas flow tube 184; a vortex guide 186; and a pair of outer magnets 188 for electronic excitation of the swirling gas in the gas flow tube 184. Each of the outer magnets 188 may have a cylindrical shell having a semicircular cross section disposed around the outer surface of the gas flow tube 184.

FIG. 6D shows a cross-sectional view of a nozzle 190 which is yet another alternative embodiment of the nozzle 36 shown in FIG. 4C. As illustrated, the nozzle 190 includes: a rod-shaped conductor 192; a gas flow tube 194; a vortex guide 196; and a pair of inner magnets 198, secured by the vortex guide 196 within the gas flow tube 194, for electronic excitation of the helical swirl in the gas flow tube 194. Each of the inner magnets 198 may have a cylindrical shell having a semicircular cross section.

FIG. 6E shows a cross-sectional view of a nozzle 200 which is a further alternative embodiment of the nozzle 36 shown in FIG. 4C. As illustrated, the nozzle 200 includes: a rod-shaped conductor 202; a gas flow tube 204; a vortex guide 206; a pair of outer magnets 208; and an inner shield 210. Each of the outer magnets 208 may have a cylindrical shell having a semicircular cross section. In an alternative embodiment, the inner shield 210 may have a tubular shape.

FIG. 6F is a cross-sectional view of a nozzle 212 which is another alternative embodiment of the nozzle 36 shown in FIG. 4C. As illustrated, the nozzle 212 includes: a rod-shaped conductor 214; a gas flow tube 216; a vortex guide 218; an anode 220; and a cathode 222. The anode 220 and the cathode 222, connected to an electrical power source (not shown in FIG. 5F for simplicity), may electronically excite the swirling gas in the gas flow tube 216.

As mentioned above, FIGS. 6A-6F show cross-sectional views of various embodiments of the nozzle 36 shown in FIG. 4B. However, it should be apparent to one of ordinary skill that the embodiments shown in FIGS. 6A-6F can be applied to the nozzles shown in FIGS. 4C-4D and 5B-5D. Also, one skilled in the art will appreciate that the descriptions in FIGS. 4A-6F may be equally applied to the system 70 in FIG. 3.

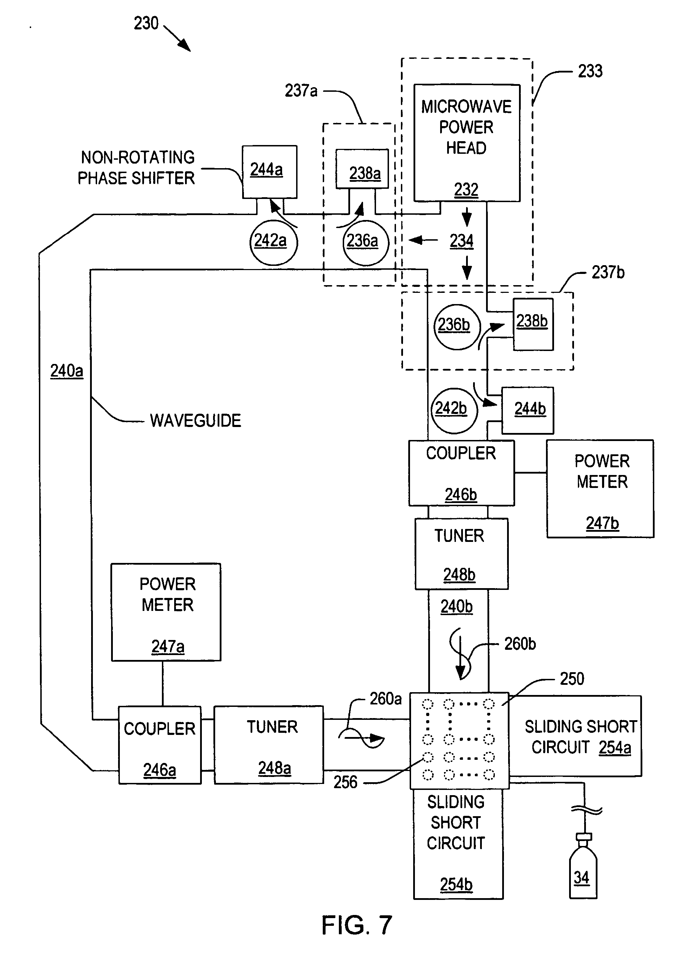

Referring back to FIG. 2B, the nozzles 36 may be configured within the high-energy regions 69 to maximize the use of microwave energy within the microwave cavity 32. In general, operational efficiency of the microwave cavity 32 may increase if the high-energy regions 69 are confined only around the nozzles 36. As the cross section of a typical nozzle is circular or rectangular with an aspect ratio of a near unity, operational efficiency of the microwave cavity may be maximized if the high-energy regions are confined within rectangular regions in a 2-dimensional matrix form as will be described in FIGS. 7-9.

FIG. 7 is a schematic diagram of a system shown at 230 having a plasma nozzle array in accordance with one embodiment of the present invention. The components of the system shown at 230 are similar to their counterparts of FIG. 1, except that the microwaves are traveling normal to each other in a microwave cavity 250. As illustrated, the system 230 includes: a microwave source 233 that has a microwave power head 232 and a power splitter 234 having two outlets; a pair of non-rotating phase shifters 244a and 244b; a pair of isolators 237a and 237b including a pair of circulators 236a and 236b and a pair of dummy loads 238a and 238b; a pair of circulators 242a and 242b; waveguides 240a and 240b; the microwave cavity 250; one or more nozzles 256, preferably forming a two-dimensional array; and a pair of sliding short circuits 254a and 254b. Inset diagrams 260a and 260b represent microwaves transmitted to the microwave cavity 250. The system 230 may further include: a pair of couplers 246a and 246b; a pair of tuners 248a and 248b; and a pair of power meters 247a and 247b connected to a pair of couplers 246a and 246b, respectively. The gas tank 34 may be connected to the microwave cavity 250 to provide a gas to the nozzles 256 that are coupled to the microwave cavity 250. In an alternative embodiment, an isolator may be located between the microwave power head 232 and the power splitter 234, replacing the isolators 237a and 237b.

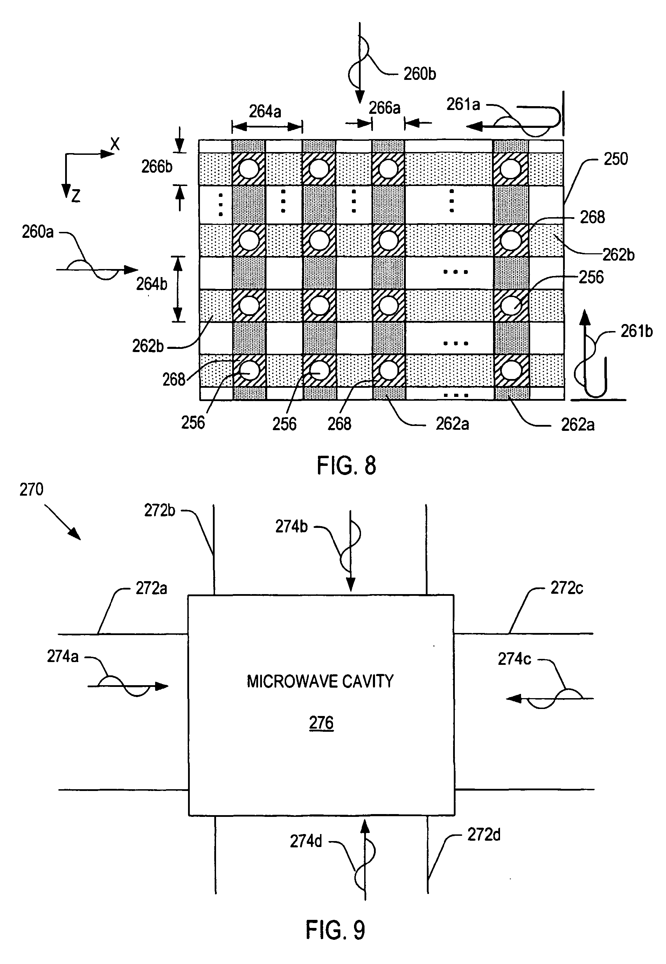

FIG. 8 illustrates a distribution of high-energy regions within the microwave cavity 250 viewed in a direction normal to a plane defined by the propagation directions of two interfering microwaves, wherein the two microwaves are shown by waveforms 260a and 260b. As shown in FIG. 8, two microwaves, shown by the waveforms 260a and 260b, and two reflected microwaves, shown by waveforms 261a and 261b, generate high-energy regions 268 in a two-dimensional array form, where intervals 264a and 264b correspond to half-wavelengths of the microwaves 260a and 260b, respectively. By the same principle as applied to the interference pattern shown in FIG. 2B, the microwaves 260a and 261 a, and the microwaves 260b and 261b, generate two standing microwaves that yield strip-shaped high-energy regions 262a and 262b, respectively. Then, the standing microwaves may further interfere to generate high-energy regions 268 in a matrix form as depicted in FIG. 8. Locations and widths 266a and 266b of the high-energy regions 258 may be controlled by the non-rotating phase shifters 244a and 244b and/or the sliding short circuits 254a and 254b. A portion of the rod-shaped conductor of each nozzle 256 may be located within the high-energy regions to collect the microwave energy, as illustrated in FIG. 8.

In an alternative embodiment, two separate microwave power heads may replace the microwave source 233, where each microwave power head may transmit microwaves to the microwave cavity 250. In such embodiment, two microwaves may have different wavelengths and amplitudes, and as a consequence, the intervals 264a and 264b may be different from each other. Likewise, the widths 266a and 266b of the high-energy regions may be different from each other.

FIG. 9 is a schematic diagram of a microwave cavity and waveguides, collectively shown at 270, for generating high-energy regions in a two-dimensional array form in accordance with still another embodiment of the present invention. As illustrated, a microwave cavity 276 may receive four microwaves 274a to 274d traveling through four waveguides 272a to 272d, respectively. The phases of the microwaves may be controlled by a corresponding one of four non-rotating phase shifters (not shown in FIG. 9) coupled to the waveguides 272a to 272d, respectively. The four microwaves 274a to 274d may be generated by one or more microwave power heads. Each of four microwaves 274a to 274d may be generated by a corresponding one of the four microwave power heads, respectively. In an alternative embodiment, two microwave power heads generate microwaves, where each microwave is split into two microwaves. In another alternative embodiment, one microwave power head may be split into four microwaves using a power splitter having four outlets. It is noted that these three embodiments are provided for exemplary purposes only. Thus, it should be apparent to those of ordinary skill that any suitable system with the capability of providing four microwaves may be used with the microwave waveguides 272a to 272d without deviating from the present invention.

Various embodiments of nozzles in FIGS. 6A-6F and walls of microwave cavities in FIG. 4B-4D that form gas flow channels may be also applied to the systems described in FIG. 9. For simplicity, such embodiments have not been shown.

Referring back to FIG. 8, the intervals 264a and 264b between two neighboring nozzles in x- and z-directions may be half wavelengths of the microwaves shown by the waveforms 260a and 260b, respectively. In some applications, these half-wavelength intervals may introduce fluctuations in plasma characteristics along the x- and z-directions and, as a consequence, smaller intervals may be required. For example, FIG. 10 schematically shows an alternative interference pattern of the high-energy regions found within the microwave cavity of the systems depicted in FIGS. 7 and 9. As illustrated, each high-energy region 268' may contain more than one nozzle 256' providing smaller intervals between neighboring nozzles. By reducing the intervals, the nozzle array coupled to the microwave cavity 250' may be able to generate a plasma having an enhanced uniformity in both x- and z-directions. As in the case of FIG. 8, the width 266a' of each high energy region 268' may be controlled by adjusting the phase difference between two microwaves 260a' and 261a', while the width 266b' may be controlled by adjusting the phase difference between two microwaves 260b' and 261b'.

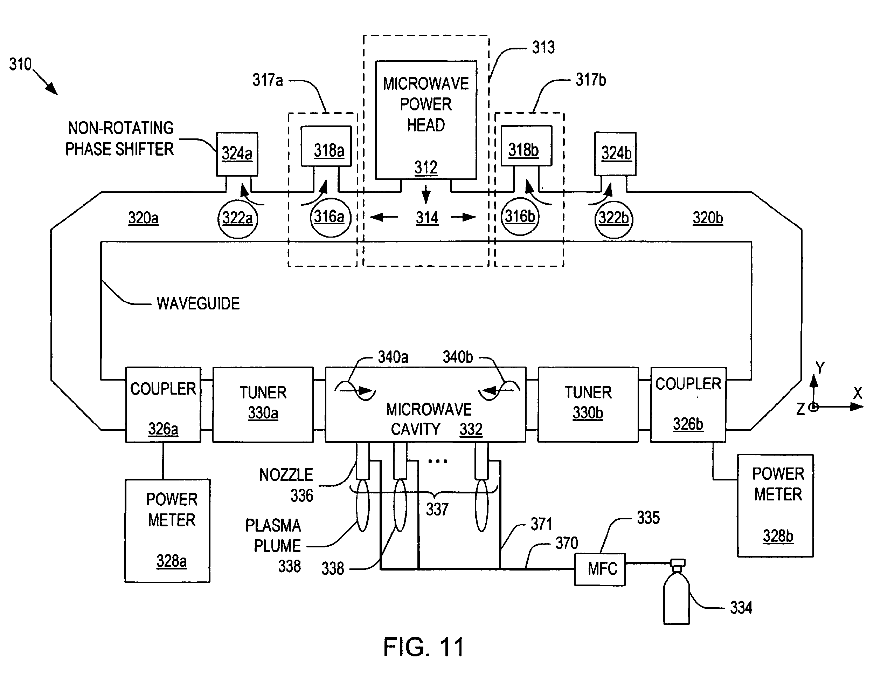

FIG. 11 is a schematic diagram of a system shown at 310 and having a plasma nozzle array 337 in accordance with still another embodiment of the present invention. As illustrated, the system shown at 310 is quite similar to the system shown at 10 (FIG. 1) with the difference that nozzles 336 in a nozzle array 337 may receive gas directly from a gas tank 334. The gas line 370 from the gas tank 334 may have a plurality of branches 371, wherein each branch may be coupled to one of the nozzles 336 and formed of a conventional gas tube.

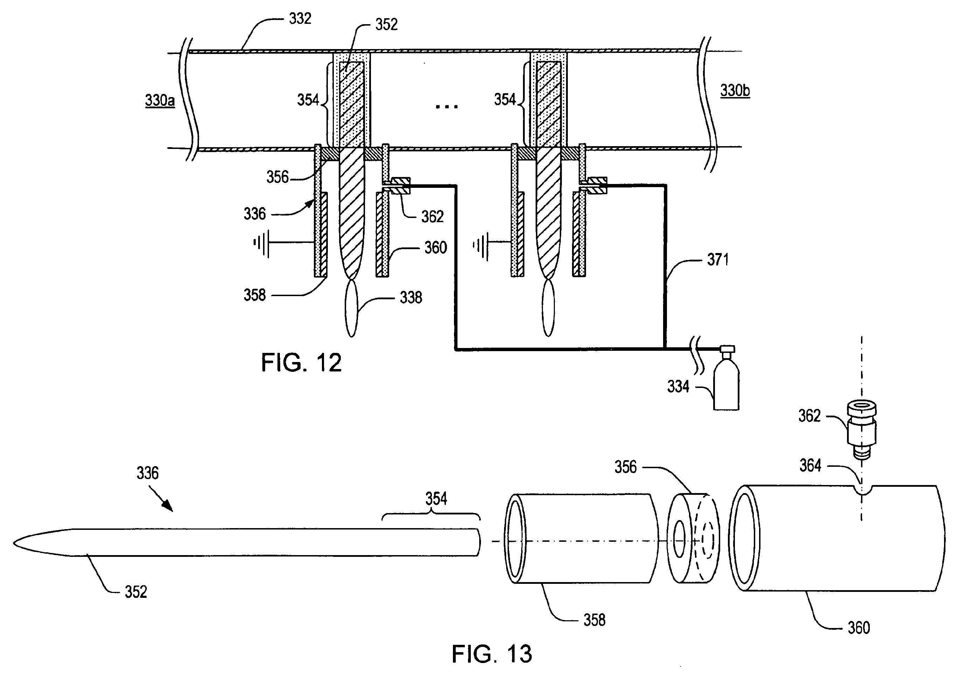

FIG. 12 shows a cross-sectional view of the microwave cavity 332 and nozzle array 337 taken along a direction normal to the z-axis in FIG. 11. As illustrated, a nozzle 336 may includes: a gas flow tube 358; a grounded shield 360 for reducing microwave loss through gas flow tube 358 and sealed with the cavity wall 332, the gas flow tube 358 being tightly fitted into the grounded shield 360; a rod-shaped conductor 352 having a portion 354 disposed in the microwave cavity 332 for receiving microwaves from within the microwave cavity 332; a position holder 356 disposed between the rod-shaped conductor 352 and the grounded shield 360 and configured to securely hold the rod-shaped conductor 352 relative to the ground shield 360; and a gas feeding mechanism 362 for coupling the branch 371 to the grounded shield 360. The position holder 356, grounded shield 360 and rod-shaped conductor 352 may be made of the same materials as those of the vortex guide 146 (FIG. 4D), grounded shield 178 (FIG. 6B) and rod-shaped conductor 152 (FIG. 4D), respectively. For example, the grounded shield 360 may be made of metal and preferably copper.

As illustrated in FIG. 12, the nozzle 336 may receive gas through the gas feeding mechanism 362. The gas feeding mechanism 362 may be a pneumatic one-touch fitting (model No. KQ2H05-32) made by SMC Corporation of America, Indianapolis, IN. One end of the gas feeding mechanism 362 has a threaded bolt that mates with the female threads formed on the edge of a hole 364 in the grounded shield 360 as illustrated in FIG. 13. It should be apparent to those of ordinary skill that the present invention may be practiced with other suitable types of gas feeding mechanisms. Several embodiments of the nozzles 336 and the microwave cavity 332 that may be used for the system 310 are discussed in the previously referred PCT Application entitled "Microwave Plasma Nozzle with Enhanced Plume Stability and Heating Efficiency," filed on July 7, 2005.

FIG. 13 is an exploded perspective view of the nozzle 336 shown in FIG. 12. As illustrated, the rod-shaped conductor 352 and the grounded shield 360 can engage the inner and outer perimeters of the position holder 356, respectively. The rod-shaped conductor 352 may have a portion 354 that acts as an antenna to collect microwaves from the microwave cavity 332. The collected microwave may travel along the rod-shaped conductor 352 and generate plasma 338 using the gas flowing through the gas flow tube 358. The term rod-shaped conductor is intended to cover conductors having various cross sections such as circular, oval, elliptical, or an oblong cross section, or any combinations thereof.

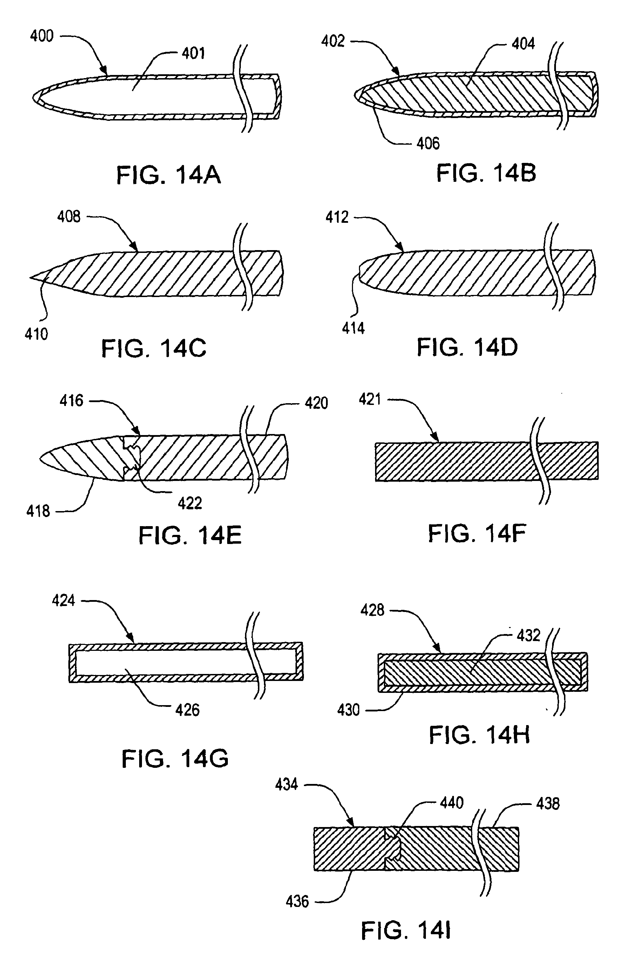

The microwaves may be collected by the portion 354 of the rod-shaped conductor 352 that extends into the microwave cavity 332. These microwaves travel down the rod-shaped conductor toward the tapered tip. More specifically, the microwaves are received by and travel along the surface of the rod-shaped conductor 352. The depth of the skin responsible for microwave penetration and migration is a function of the microwave frequency and the conductor material. The microwave penetration distance can be less than a millimeter. Thus, a rod-shaped conductor 400 of FIG. 14A having a hollow portion 401 is an alternative embodiment for the rod-shaped conductor 352.

It is well known that some precious metals are good microwave conductors. Thus, to reduce the unit price of the device without compromising the performance of the rod-shaped conductor, the skin layer of the rod-shaped conductor can be made of precious metals that are good microwave conductors while cheaper conducting materials can be used for inside of the core. FIG. 14B is a cross-sectional view of another alternative embodiment of a rod-shaped conductor, wherein a rod-shaped conductor 402 includes skin layer 406 made of a precious metal and a core layer 404 made of a cheaper conducting material.

FIG. 14C is a cross-sectional view of yet another alternative embodiment of the rod-shaped conductor, wherein a rod-shaped conductor 408 includes a conically-tapered tip 410. Other cross-sectional variations can also be used. For example, conically-tapered tip 410 may be eroded by plasma faster than another portion of rod-conductor 408 and thus may need to be replaced on a regular basis.

FIG. 14D is a cross-sectional view of another alternative embodiment of the rod-shaped conductor, wherein a rod-shaped conductor 412 has a blunt-tip 414 instead of a pointed tip to increase the lifetime thereof.

FIG. 14E is a cross-sectional view of another alternative embodiment of the rod-shaped conductor, wherein a rod-shaped conductor 416 has a tapered section 418 secured to a cylindrical portion 420 by a suitable fastening mechanism 422 (in this case, the tapered section 418 can be screwed into the cylindrical portion 420 using the screw end 422) for easy and quick replacement thereof.

FIGS. 14F-14I show cross-sectional views of further alternative embodiments of the rod-shaped conductor. As illustrated, rod-shaped conductors 421, 424, 428 and 434 are similar to their counterparts 352 (FIG. 13), 400 (FIG. 14A), 402 (FIG. 14B) and 416 (FIG. 14E), respectively, with the difference that they have blunt tips for reducing the erosion rate due to plasma. It is noted that the various embodiments of rod-shaped conductor depicted in FIGS. 14A-14I can be used in any embodiment of the nozzle described in FIGS. 1 and 3-13.

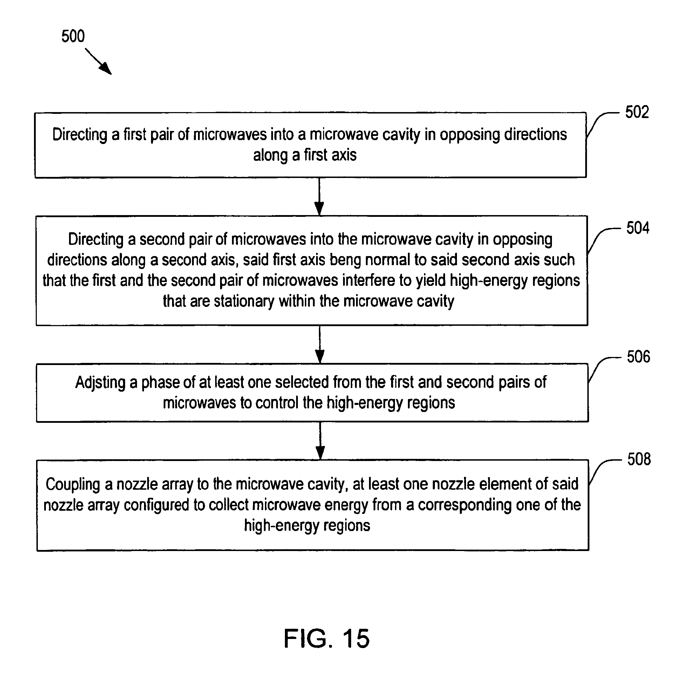

FIG. 15 shows a flowchart 500 illustrating exemplary steps for configuring a microwave plasma nozzle array in accordance with at least one embodiment of the present invention. At step 502, the first pair of microwaves is directed into a microwave cavity in opposing directions along a first axis. Next, at step 504, the second pair of microwaves is directed into the microwave cavity in opposing directions along a second axis, where the first axis is normal to the second axis such that the first and the second pairs of microwaves interfere to yield high-energy regions that are stationary within the microwave cavity. Then, a phase of at least one microwave selected from the first and second pair of microwaves is adjusted to control the high-energy regions at step 506. Finally, at step 508, a nozzle array is coupled to the microwave cavity, where one or more nozzle elements of the nozzle array are configured to collect the microwave energy from a corresponding one of the high-energy regions.

While the present invention has been described with a reference to the specific embodiments thereof, it should be understood, of course, that the foregoing relates to preferred embodiments of the invention and the scope of the invention is defined in the following claims.

In addition, many modifications may be made to adapt a particular situation, systems, process, process step or steps, to the objective and the scope of the present invention. All such modifications are intended to be within the scope of the claims appended hereto.

## Claims

1. A method for configuring a microwave plasma nozzle array, comprising the steps of:
directing microwaves into a microwave cavity (32, 92, 113, 113', 133, 142, 250, 276, 332) in opposing directions such that the microwaves interfere and form a standing microwave pattern that is stationary within the microwave cavity (32, 92, 113, 113', 133, 142, 250, 276, 332);
adjusting a phase of at least one of the microwaves to control high-energy regions (69, 69', 258, 262a, 262b 268, 268') generated by the standing microwave pattern; and
disposing a nozzle array (37, 99, 337) at least partially in the microwave cavity (32, 92, 113, 113', 133, 142, 250, 276, 332) so that one or more nozzle elements (36, 36', 98, 128, 144, 160, 170, 180, 190, 200, 212, 256, 256', 336) of the nozzle array (37, 99, 337) are configured to receive microwave energy from a corresponding one of the high-energy regions (69, 69', 258, 262a, 262b 268, 268'), wherein
each of said nozzles (36, 36', 98, 128, 144, 160, 170, 180, 190, 200, 212, 256, 256', 336) includes a gas flow tube (120, 134, 148, 164, 174, 184, 194, 204, 216, 358) adapted to direct a gas flow therethrough and having an inlet portion and an outlet portion,
said outlet portion being provided outside of said microwave cavity (32, 92, 113, 113', 133, 142, 250, 276, 332), **characterized in that** each of said nozzles further includes
- a rod-shaped conductor (114, 130, 152, 162, 172, 182, 192, 202, 214, 352, 400, 408, 412, 416, 421, 424, 428, 434) axially disposed in said gas flow tube (120, 134, 148, 164, 174, 184, 194, 204, 216, 358), said rod-shaped conductor (114, 130, 152, 162, 172, 182, 192, 202, 214, 352, 400, 408, 412, 416, 421, 424, 428, 434) having a tip (117, 410, 414) and a collection portion (116, 354), which is disposed in said microwave cavity (32, 92, 113, 113', 133, 142, 250, 276, 332) to receive microwaves at said collection portion (116, 354) and transmits the received microwaves along the surface thereof and focuses the microwaves at said tip (117, 410, 414) and wherein said tip (117, 410, 414) is positioned adjacent said outlet portion of said gas flow tube (120, 134, 148, 164, 174, 184, 194, 204, 216, 358).

2. A method as defined in claim 1, wherein said step of directing microwaves includes the steps of:
transmitting microwaves to the microwave cavity (32, 92, 113, 113', 133, 142, 250, 276, 332); and
reflecting microwaves using a sliding short circuit (94, 254a, 254b) operatively connected to the microwave cavity (32, 92, 113, 113', 133, 142, 250, 276, 332).

3. A method as defined in claim 1, wherein said step of directing microwaves includes the step of:
transmitting microwaves generated by two microwave power heads (12, 72, 232) to the microwave cavity (32, 92, 113, 113', 133, 142, 250, 276, 332).

4. A method as defined in claim 1, comprising the steps of:
directing a second pair of microwaves into the microwave cavity (32, 92, 113, 113', 133, 142, 250, 276, 332) in opposing directions along a second axis, the first axis being normal to the second axis such that the first and the second pairs of microwaves interfere and form high-energy regions (69, 69', 258, 262a, 262b 268, 268') that are stationary within the microwave cavity (32, 92, 113, 113', 133, 142, 250, 276, 332).

5. A method as defined in claim 4, wherein said step of directing the first pair of microwaves includes the steps of:
transmitting microwaves to the microwave cavity (32, 92, 113, 113', 133, 142, 250, 276, 332); and
reflecting microwaves using a sliding short circuit (94, 254a, 254b) operatively connected the microwave cavity (32, 92, 113, 113', 133, 142, 250, 276, 332).

6. A method as defined in claim 4, wherein said step of directing the first pair of microwaves includes the step of:
transmitting microwaves generated by two microwave power heads (12, 72, 232) to the microwave cavity (32, 92, 113, 113', 133, 142, 250, 276, 332).

7. A method as defined in claim 4, further comprising the steps of:
generating the microwaves by a microwave power head (12, 72, 232); and providing a power splitter (14, 234) connected to the microwave power head (12, 72, 232).

8. A method as defined in claim 4, wherein said step of adjusting a phase of at least one of the microwaves includes adjusting phases of the first pair of microwaves.

9. A method as defined in claim 4, wherein said step of adjusting a phase of at least one of the microwaves includes adjusting phases of the second pair of microwaves.

10. A method as defined in claim 4, wherein said step of adjusting a phase of at least one of the microwaves includes adjusting phases of both the first pair and the second pair of microwaves.

11. A microwave plasma nozzle array unit, comprising:
a microwave cavity (32, 92, 113, 113', 133, 142, 250, 276, 332); and an array of nozzles (37, 99, 337), at least partially disposed in the microwave cavity of said nozzles (36, 36', 98, 128, 144, 160, 170, 180, 190, 200, 212, 256, 256', 336) including:
a gas flow tube (120, 134, 148, 164, 174, 184, 194, 204, 216, 358) adapted to direct a gas flow therethrough and having an inlet portion and an outlet portion, said outlet portion being provided outside of said microwave cavity (32, 92, 113, 113', 133, 142, 250, 276, 332); and
a rod-shaped conductor (114, 130, 152, 162, 172, 182, 192, 202, 214, 352, 400, 408, 412, 416, 421, 424, 428, 434) axially disposed in said gas flow tube (120, 134, 148, 164, 174, 184, 194, 204, 216, 358), said rod-shaped conductor (114, 130, 152, 162, 172, 182, 192, 202, 214, 352, 400, 408, 412, 416, 421, 424, 428, 434) having a tip (117, 410, 414) and a collection portion (116, 354), which is disposed in said microwave cavity (32, 92, 113, 113', 133, 142, 250, 276, 332) to receive microwaves at said collection portion (116, 354) and transmits the received microwaves and
wherein said tip (117, 410, 414) is positioned adjacent said outlet portion of said gas flow tube (120, 134, 148, 164, 174, 184, 194, 204, 216, 358).

12. A microwave plasma nozzle array unit as defined in claim 11, wherein each of said nozzles (36, 36', 98, 128, 144, 160, 170, 180, 190, 200, 212, 256, 256', 336) further includes:
a vortex guide (118, 132, 146, 166, 176 186, 196, 206, 218) disposed between said rod-shaped conductor (114, 130, 152, 162, 172, 182, 192, 202, 214, 352, 400, 408, 412, 416, 421, 424, 428, 434) and said gas flow tube (120, 134, 148, 164, 174, 184, 194, 204, 216, 358), said vortex guide (118, 132, 146, 166, 176 186, 196, 206, 218) having at least one passage for imparting a helical shaped flow direction around said rod-shaped conductor (114, 130, 152, 162, 172, 182, 192, 202, 214, 352, 400, 408, 412, 416, 421, 424, 428, 434) to a gas passing along said at least one passage.

13. A microwave plasma nozzle array unit as defined in claim 12, wherein said microwave cavity (32, 92, 113, 113', 133, 142, 250, 276, 332) includes a wall (111, 143, 332), said wall (111, 143, 332) of said microwave cavity (32, 92, 113, 113', 133, 142, 250, 276, 332) forming a portion of a gas flow passage (112, 127) operatively connected to the inlet portion of said gas flow tube (120, 134, 148, 164, 174, 184, 194, 204, 216, 358).

14. A microwave plasma nozzle array unit as defined in claim 11, wherein each of said nozzles (36, 36', 98, 128, 144, 160, 170, 180, 190, 200, 212, 256, 256', 336) further includes:
a shield (168, 210) disposed adjacent to a portion of said gas flow tube (120, 134, 148, 164, 174, 184, 194, 204, 216, 358) for reducing a microwave power loss through said gas flow tube (120, 134, 148, 164, 174, 184, 194, 204, 216, 358), said shield (168, 210) being made of a conducting material.

15. A microwave plasma nozzle array unit as defined in claim 11, wherein each of said nozzles (36, 36', 98, 128, 144, 160, 170, 180, 190, 200, 212, 256, 256', 336) further includes:
a grounded shield (178, 360) disposed on an exterior surface of said gas flow tube (120, 134, 148, 164, 174, 184, 194, 204, 216, 358) for reducing a microwave power loss through said gas flow tube (120, 134, 148, 164, 174, 184, 194, 204, 216, 358), said grounded shield (178, 360) having a hole for receiving the gas flow therethrough.

16. A microwave plasma nozzle array unit as defined in claim 15, wherein each of said nozzles (36, 36', 98, 128, 144, 160, 170, 180, 190, 200, 212, 256, 256', 336) further includes:
a position holder (356) disposed between said rod-shaped conductor (114, 130, 152, 162, 172, 182, 192, 202, 214, 352, 400, 408, 412, 416, 421, 424, 428, 434) and said grounded shield (178, 360) for securely holding said rod-shaped conductor (114, 130, 152, 162, 172, 182, 192, 202, 214, 352, 400, 408, 412, 416, 421, 424, 428, 434) relative to said grounded shield (178, 360).

17. A microwave plasma nozzle array unit as defined in claim 11, wherein said gas flow tube (120, 134, 148, 164, 174, 184, 194, 204, 216, 358) is made of quartz.

18. A microwave plasma nozzle array unit as defined in claim 11, wherein each of said nozzles (36, 36', 98, 128, 144, 160, 170, 180, 190, 200, 212, 256, 256', 336) further includes a pair of magnets (188, 198, 208) disposed adjacent to said gas flow tube (120, 134, 148, 164, 174, 184, 194, 204, 216, 358), said pair of magnets (188, 198, 208) having a shape approximating a portion of a cylinder.

19. A microwave plasma nozzle array unit as defined in claim 11, wherein each of said nozzles (36, 36', 98, 128, 144, 160, 170, 180, 190, 200, 212, 256, 256', 336) further includes:
an anode (220) disposed adjacent to a portion of said gas flow tube (120, 134, 148, 164, 174, 184, 194, 204, 216, 358); and
a cathode (222) disposed adjacent to another portion of said gas flow tube (120, 134, 148, 164, 174, 184, 194, 204, 216, 358).

20. A microwave plasma nozzle array unit as defined in claim 11, wherein said microwave cavity (32, 92, 113, 113', 133, 142, 250, 276, 332) includes:
a microwave inlet; and
a sliding short circuit (94, 254a, 254b) configured to reflect microwaves transmitted through said microwave inlet.

21. A microwave plasma nozzle array unit as defined in claim 11, wherein said microwave cavity (32, 92, 113, 113', 133, 142, 250, 276, 332) includes:
two microwave inlets disposed in opposite sides of said microwave cavity (32, 92, 113, 113', 133, 142, 250, 276, 332).

22. A microwave plasma nozzle array unit as defined in claim 11, wherein said microwave cavity (32, 92, 113, 113', 133, 142, 250, 276, 332) includes:
two microwave inlets disposed in sides of said microwave cavity (32, 92, 113, 113', 133, 142, 250, 276, 332) which are normal to each other; and
two sliding short circuits (94, 254a, 254b) configured to reflect microwaves received by said inlets.

23. A microwave plasma nozzle array unit as defined in claim 11, wherein said microwave cavity (32, 92, 113, 113', 133, 142, 250, 276, 332) includes:
a first pair of microwave inlets disposed in opposite sides of said microwave cavity (32, 92, 113, 113', 133, 142, 250, 276, 332) along a first axis;
a second pair of microwave inlets disposed in opposite sides of said microwave cavity (32, 92, 113, 113', 133, 142, 250, 276, 332) along a second axis, the second axis being substantially normal to the first axis.

24. A microwave plasma nozzle array unit as defined in claim 11, wherein said microwave cavity (32, 92, 113, 113', 133, 142, 250, 276, 332) is configured to generate a plurality of stationary high-energy regions (69, 69', 258, 262a, 262b 268, 268') using microwaves directed thereto and wherein said portion of said rod-shaped conductor (114, 130, 152, 162, 172, 182, 192, 202, 214, 352, 400, 408, 412, 416, 421, 424, 428, 434) is disposed within the space occupied by said stationary high-energy regions (69, 69', 258, 262a, 262b 268, 268').

25. A microwave plasma system, comprising:
a microwave plasma nozzle unit according to any one of the claims 11 to 24;
an isolator (17a, 17b, 74, 237a, 237b) operatively connected to said microwave source (13, 233);
a microwave cavity (32, 92, 113, 113', 133, 142, 250, 276, 332) having an inlet;
a waveguide (20a, 20b, 90, 272a-d) operatively connected to said isolator (17a, 17b, 74, 237a, 237b) and to said inlet of said microwave cavity (32, 92, 113, 113', 133, 142, 250, 276, 332);
a non-rotating phase shifter (24a, 24b, 82, 244a, 244b) operatively connected to said waveguide (20a, 20b, 90, 272a-d) and said isolator (17a, 17b, 74, 237a, 237b);
a circulator (16, 22a, 22b, 78, 80, 236a, 236b, 242a, 242b) operatively connected to said waveguide (20a, 20b, 90, 272a-d) and configured to direct microwaves to said non-rotating phase shifter (24a, 24b, 82, 244a, 244b); and
a sliding short circuit (94, 254a, 254b) operatively connected to said microwave cavity (32, 92, 113, 113', 133, 142, 250, 276, 332).

26. A microwave plasma system as defined in claim 25, wherein each of said nozzles (36, 36', 98, 128, 144, 160, 170, 180, 190, 200, 212, 256, 256', 336) further includes:
a vortex guide (118, 132, 146, 166, 176 186, 196, 206, 218) disposed between said rod-shaped conductor (114, 130, 152, 162, 172, 182, 192, 202, 214, 352, 400, 408, 412, 416, 421, 424, 428, 434) and said gas flow tube (120, 134, 148, 164, 174, 184, 194, 204, 216, 358), said vortex guide (118, 132, 146, 166, 176 186, 196, 206, 218) having at least one passage for imparting a helical shaped flow direction around said rod-shaped conductor ( 114, 130, 152, 162, 172, 182, 192, 202, 214, 352, 400, 408, 412, 416, 421, 424, 428, 434) to a gas passing along said at least one passage.

27. A microwave plasma system as defined in claim 26, wherein said microwave cavity (32, 92, 113, 113', 133, 142, 250, 276, 332) includes a wall (111, 143, 332), said wall (111, 143, 332) of said microwave cavity (32, 92, 113, 113', 133, 142, 250, 276, 332) forming a portion of a gas flow passage (112, 127) operatively connected to the inlet portion of said gas flow tube (120, 134, 148, 164, 174, 184, 194, 204, 216, 358).

28. A microwave plasma system as defined in claim 25, wherein each of said nozzles (36, 36', 98, 128, 144, 160, 170, 180, 190, 200, 212, 256, 256', 336) further includes:
a shield (168, 210) disposed adjacent to a portion of said gas flow tube (120, 134, 148, 164, 174, 184, 194, 204, 216, 358) for reducing a microwave power loss through said gas flow tube (120, 134, 148, 164, 174, 184, 194, 204, 216, 358), said shield (168, 210) being made of a conducting material.

29. A microwave plasma system as defined in claim 25, wherein each of said nozzles (36, 36', 98, 128, 144, 160, 170, 180, 190, 200, 212, 256, 256', 336) further includes:
a grounded shield (178, 360) disposed on an exterior surface of said gas flow tube (120, 134, 148, 164, 174, 184, 194, 204, 216, 358) for reducing a microwave power loss through said gas flow tube (120, 134, 148, 164, 174, 184, 194, 204, 216, 358), said grounded shield (178, 360) having a hole for receiving the gas flow therethrough.

30. A microwave plasma system as defined in claim 29, wherein each of said nozzles (36, 36', 98, 128, 144, 160, 170, 180, 190, 200, 212, 256, 256', 336) further includes:
a position holder (356) disposed between said rod-shaped conductor (114, 130, 152, 162, 172, 182, 192, 202, 214, 352, 400, 408, 412, 416, 421, 424, 428, 434) and said grounded shield (178, 360) for securely holding said rod-shaped conductor (114, 130, 152, 162, 172, 182, 192, 202, 214, 352, 400, 408, 412, 416, 421, 424, 428, 434) relative to said grounded shield (178, 360).

31. A microwave plasma system as defined in claim 25, wherein said gas flow tube (120, 134, 148, 164, 174, 184, 194, 204, 216, 358) is made of quartz.

32. A microwave plasma system as defined in claim 25, wherein each of said nozzles (36, 36', 98, 128, 144, 160, 170, 180, 190, 200, 212, 256, 256', 336) further includes a pair of magnets (188, 198, 208) disposed adjacent to said gas flow tube (120, 134, 148, 164, 174, 184, 194, 204, 216, 358), said pair of magnets (188, 198, 208) having a shape approximating a portion of a cylinder.

33. A microwave plasma system as defined in claim 25, wherein each of said nozzles (36, 36', 98, 128, 144, 160, 170, 180, 190, 200, 212, 256, 256', 336) further includes:
an anode (220) disposed adjacent to a portion of said gas flow tube (120, 134, 148, 164, 174, 184, 194, 204, 216, 358); and
a cathode (222) disposed adjacent to another portion of said gas flow tube (120, 134, 148, 164, 174, 184, 194, 204, 216, 358).

34. A microwave plasma system as defined in claim 25, wherein said microwave cavity (32, 92, 113, 113', 133, 142, 250, 276, 332) is configured to generate a plurality of stationary high-energy regions (69, 69', 258, 262a, 262b 268, 268') using microwaves directed thereto and wherein said portion (116, 354) of said rod-shaped conductor (114, 130, 152, 162, 172, 182, 192, 202, 214, 352, 400, 408, 412, 416, 421, 424, 428, 434) is disposed within the space occupied by said stationary high-energy regions (69, 69', 258, 262a, 262b 268, 268').

35. A microwave plasma system as defined in claim 25, wherein said isolator (17a, 17b, 74, 237a, 237b) includes:
a circulator (16, 22a, 22b, 78, 80, 236a, 236b, 242a, 242b) operatively connected to said waveguide (20a, 20b, 90, 272a-d); and
a dummy load (18a, 18b, 76, 238a, 238b) operatively connected to said circulator (16, 22a, 22b, 78, 80, 236a, 236b, 242a, 242b).

36. A microwave plasma system as defined in claim 25, further comprising:
a tuner (30a, 30b, 88, 248a, 248b) operatively connected to said waveguide (20a, 20b, 90, 272a-d) and said microwave cavity (32, 92, 113, 113', 133, 142, 250,276,332).

37. A microwave plasma system as defined in claim 25, further comprising:
a coupler (26a, 26b, 86, 246a, 246b) operatively connected to said waveguide (20a, 20b, 90, 272a-d) and a power meter (28a, 28b, 84, 247a, 247b) for measuring microwave fluxes.

38. A microwave plasma system according to any one of claims 25 to 37, comprising:
a pair of isolators (17a, 17b, 74, 237a, 237b);
a pair of inlets;
a pair of waveguides (20a, 20b, 90, 272a-d), each of said waveguides (20a, 20b, 90, 272a-d) being operatively connected to a corresponding one of said isolators (17a, 17b, 74, 237a, 237b) and to a corresponding one of said inlets of said microwave cavity (32, 92, 113, 113', 133, 142, 250, 276, 332); and
a pair of non-rotating phase shifters (24a, 24b, 82, 244a, 244b), each of said non-rotating phase shifters (24a, 24b, 82, 244a, 244b) being operatively connected to a corresponding one of said waveguides (20a, 20b, 90, 272a-d) and to a corresponding one of said isolators (17a, 17b, 74, 237a, 237b).

39. A microwave plasma system as defined in claim 38, further comprising:
a pair of tuners (30a, 30b, 88, 248a, 248b), each of said tuners (30a, 30b, 88, 248a, 248b) being operatively connected to a corresponding one of said waveguides (20a, 20b, 90, 272a-d) and said microwave cavity (32, 92, 113, 113', 133, 142, 250, 276, 332).

40. A microwave plasma system as defined in claim 38, further comprising:
a pair of circulators (16, 22a, 22b, 78, 80, 236a, 236b, 242a, 242b), each of said circulators (16, 22a, 22b, 78, 80, 236a, 236b, 242a, 242b) being operatively connected to a corresponding one of said waveguides (20a, 20b, 90, 272a-d) and configured to direct microwaves to a corresponding one of said non-rotating phase shifters (24a, 24b, 82, 244a, 244b).

41. A microwave plasma system as defined in claim 38, further comprising:
a pair of couplers (26a, 26b, 86, 246a, 246b), each of said couplers (26a, 26b, 86, 246a, 246b) being operatively connected to a corresponding one of said waveguides (20a, 20b, 90, 272a-d) and a power meter (28a, 28b, 84, 247a, 247b) for measuring microwave fluxes.

42. A microwave plasma system as defined in claim 38, wherein said microwave source (13, 233) includes a pair of microwave power heads (12, 72, 232), each of said microwave power heads (12, 72, 232) being operatively connected to a corresponding one of said isolators (17a, 17b, 74, 237a, 237b).

43. A microwave plasma system as defined in claim 38, wherein said microwave source (13, 233) includes:
a microwave power head (12, 72, 232) for generating microwaves; and
a power splitter (14, 234) for receiving, bisecting and directing the microwaves to said isolators (17a, 17b, 74, 237a, 237b).

44. A microwave plasma system according to claim 38, comprising:
a pair of sliding short circuits (94, 254a, 254b).

45. A microwave plasma system according to any one of claims 25 to 37, comprising:
four inlets;
four waveguides (20a, 20b, 90, 272a-d), each of said waveguides (20a, 20b, 90, 272a-d) being operatively connected to a corresponding one of said inlets of said microwave cavity (32, 92, 113, 113', 133, 142, 250, 276, 332) and said microwave source (13, 233);
four non-rotating phase shifters (24a, 24b, 82, 244a, 244b), each of said non-rotating phase shifters (24a, 24b, 82, 244a, 244b) being operatively connected to a corresponding one of said waveguides (20a, 20b, 90, 272a-d) and said microwave source (13, 233);
four circulators (16, 22a, 22b, 78, 80, 236a, 236b, 242a, 242b), each of said circulators (16, 22a, 22b, 78, 80, 236a, 236b, 242a, 242b) being operatively connected to a corresponding one of said waveguides (20a, 20b, 90, 272a-d) and configured to direct microwaves generated by said microwave source (13, 233) to at least one of said non-rotating phase shifters (24a, 24b, 82, 244a, 244b).

46. A microwave plasma system as defined in claim 45, wherein said microwave source (13, 233) includes:
four microwave power heads (12, 72, 232); and
four isolators (17a, 17b, 74, 237a, 237b), each of said isolators (17a, 17b, 74, 237a, 237b) being operatively connected to a corresponding one of said microwave power heads (12, 72, 232) and to at least one of said waveguides (20a, 20b, 90, 272a-d), each of said isolators (17a, 17b, 74, 237a, 237b) including:
a circulator (16, 22a, 22b, 78, 80, 236a, 236b, 242a, 242b) operatively connected to said waveguide (20a, 20b, 90, 272a-d); and
a dummy load (18a, 18b, 76, 238a, 238b) operatively connected to said circulator (16, 22a, 22b, 78, 80, 236a, 236b, 242a, 242b).

47. A microwave plasma system as defined in claim 45, wherein said microwave source (13, 233) includes:
two microwave power heads (12, 72, 232);
two isolators (17a, 17b, 74, 237a, 237b), each of said isolators (17a, 17b, 74, 237a, 237b) being connected to a corresponding one of said microwave power heads (12, 72, 232), each of said isolators (17a, 17b, 74, 237a, 237b) including:
a circulator (16, 22a, 22b, 78, 80, 236a, 236b, 242a, 242b) operatively connected to said waveguide (20a, 20b, 90, 272a-d); and
a dummy load (18a, 18b, 76, 238a, 238b) operatively connected to said circulator (16, 22a, 22b, 78, 80, 236a, 236b, 242a, 242b); and
two power splitters (14, 234), each of said power splitters (14, 234) being operatively connected to a corresponding one of said isolators (17a, 17b, 74, 237a, 237b), each of said power splitters (14, 234) being configured for receiving, bisecting and directing the microwaves to a corresponding two of said waveguides (20a, 20b, 90, 272a-d).

48. A microwave plasma system as defined in claim 45, wherein said microwave source (13, 233) includes:
a microwave power head (12, 72, 232);
an isolator (17a, 17b, 74, 237a, 237b) operatively connected to said microwave power head (12, 72, 232), said isolator (17a, 17b, 74, 237a, 237b) including:
a circulator (16, 22a, 22b, 78, 80, 236a, 236b, 242a, 242b) operatively connected to said waveguide (20a, 20b, 90, 272a-d); and
a dummy load (18a, 18b, 76, 238a, 238b) operatively connected to said circulator (16, 22a, 22b, 78, 80, 236a, 236b, 242a, 242b); and
a power splitter (14, 234) connected to said isolator (17a, 17b, 74, 237a, 237b), said power splitter (14, 234) being configured to receive, split and direct the microwaves to a corresponding one of said waveguides (20a, 20b, 90,
272a-d).

49. A microwave plasma system as defined in claim 45, further comprising:
four tuners (30a, 30b, 88, 248a, 248b), each of said tuners (30a, 30b, 88, 248a, 248b) being operatively connected to a corresponding one of said waveguides (20a, 20b, 90, 272a-d) and said microwave cavity (32, 92, 113, 113', 133, 142, 250, 276, 332).

50. A microwave plasma system as defined in claim 45, further comprising:
four couplers (26a, 26b, 86, 246a, 246b), each of said couplers (26a, 26b, 86, 246a, 246b) being operatively connected to a corresponding one of said waveguides (20a, 20b, 90, 272a-d) and a power meter (28a, 28b, 84, 247a, 247b) for measuring microwave fluxes.

## Patentansprüche

1. Verfahren zum Aufbau einer Mikrowellen-Plasmadüsenanordnung, das die folgenden Schritte umfasst:
Leiten von Mikrowellen in einen Mikrowellenhohlraum (32, 92, 113, 113', 133, 142, 250, 276, 332) in entgegengesetzte Richtungen, so dass die Mikrowellen interferieren und ein stehendes Mikrowellenmuster bilden, das innerhalb des Mikrowellenhohlraums (32, 92, 113, 113', 133, 142, 250, 276, 332) stationär ist;
Einstellen einer Phase wenigstens einer der Mikrowellen, um Hochenergiebereiche (69, 69', 258, 262a, 262b, 268, 268') zu steuern, die von dem stehenden Mikrowellenmuster erzeugt werden; und
Anordnen einer Düsenanordnung (37, 99, 337) wenigstens teilweise in dem Mikrowellenhohlraum (32, 92, 113, 113', 133, 142, 250, 276, 332), so dass ein oder mehrere Düsenelemente (36, 36', 98, 128, 144, 160, 170, 180, 190, 200, 212, 256, 256', 336) der Düsenanordnung (37, 99, 337) aufgebaut sind, um Mikrowellenenergie von einem entsprechenden der Hochenergiebereiche (69, 69', 258, 262a, 262b, 268, 268') zu empfangen, wobei
jede der Düsen (36, 36', 98, 128, 144, 160, 170, 180, 190, 200, 212, 256, 256', 336) eine Gasströmungsröhre (120, 134, 148, 164, 174, 184, 194, 204, 216, 358) umfasst, die geeignet ist, um eine Gasströmung hindurch zu leiten, und die einen Einlassabschnitt und einen Auslassabschnitt hat, wobei der Auslassabschnitt außerhalb des Mikrowellenhohlraums (32, 92, 113, 113', 133, 142, 250, 276, 332) bereitgestellt ist, **dadurch gekennzeichnet, dass** jede der Düsen ferner umfasst:
- einen stangenförmigen Leiter (114, 130, 152, 162, 172, 182, 192, 202, 214, 352, 400, 408, 412, 416, 421, 424, 428, 434), der axial in der Gasströmungsröhre (120, 134, 148, 164, 174, 184, 194, 204, 216, 358) angeordnet ist, wobei der stangenförmige Leiter (114, 130, 152, 162, 172, 182, 192, 202, 214, 352, 400, 408, 412, 416, 421, 424, 428, 434) eine Spitze (117, 410, 414) und einen Sammelabschnitt (116, 354) hat, der in dem Mikrowellenhohlraum (32, 92, 113, 113', 133, 142, 250, 276, 332) angeordnet ist, um Mikrowellen an dem Sammelabschnitt (116, 354) zu empfangen, und der die empfangenen Mikrowellen entlang seiner Oberfläche sendet und die Mikrowellen an der Spitze (117, 410, 414) fokussiert, und wobei die Spitze (117, 410, 414) benachbart zu dem Auslassabschnitt der Gasströmungsröhre (120, 134, 148, 164, 174, 184, 194, 204, 216, 358) positioniert ist.

2. Verfahren nach Anspruch 1, wobei der Schritt des Leitens der Mikrowellen, die folgenden Schritte umfasst:
Senden von Mikrowellen an den Mikrowellenhohlraum (32, 92, 113, 113', 133, 142, 250, 276, 332); und
Reflektieren von Mikrowellen unter Verwendung eines Kurzschlussschiebers (94, 254a, 254b), der betriebsfähig mit dem Mikrowellenhohlraum (32, 92, 113, 113', 133, 142, 250, 276, 332) verbunden ist.

3. Verfahren nach Anspruch 1, wobei der Schritt des Leitens von Mikrowellen den folgenden Schritt umfasst:
Senden von Mikrowellen, die von zwei Mikrowellenleistungsköpfen (12, 72, 232) erzeugt werden, an den Mikrowellenhohlraum (32, 92, 113, 113', 133, 142, 250, 276, 332).

4. Verfahren nach Anspruch 1, das die folgenden Schritte umfasst:
Leiten eines zweiten Paars von Mikrowellen in den Mikrowellenhohlraum (32, 92, 113, 113', 133, 142, 250, 276, 332) in entgegengesetzte Richtungen entlang einer zweiten Achse, wobei die erste Achse normal zu der zweiten Achse ist, so dass die ersten und die zweiten Paare von Mikrowellen interferieren und Hochenergiebereiche (69, 69', 258, 262a, 262b, 268, 268') bilden, die innerhalb des Mikrowellenhohlraums (32, 92, 113, 113', 133, 142, 250, 276, 332) stationär sind.

5. Verfahren nach Anspruch 4, wobei der Schritt des Leitens des ersten Paars von Mikrowellen, die folgenden Schritte umfasst:
Senden von Mikrowellen in den Mikrowellenhohlraum (32, 92, 113, 113', 133, 142, 250, 276, 332); und
Reflektieren von Mikrowellen unter Verwendung eines Kurzschlussschiebers (94, 254a, 254b), der betriebsfähig mit dem Mikrowellenhohlraum (32, 92, 113, 113', 133, 142, 250, 276, 332) verbunden ist.

6. Verfahren nach Anspruch 4, wobei der Schritt des Leitens des ersten Paars von Mikrowellen den folgenden Schritt umfasst:
Senden von Mikrowellen, die von zwei Mikrowellenleistungsköpfen (12, 72, 232) erzeugt werden, in den Mikrowellenhohlraum (32, 92, 113, 113', 133, 142, 250, 276, 332).

7. Verfahren nach Anspruch 4, das ferner die folgenden Schritte umfasst:
Erzeugen der Mikrowellen durch einen Mikrowellenleistungskopf (12, 72, 232); und
Bereitstellen eines Leistungsteilers (14, 234), der mit dem Mikrowellenleistungskopf (12, 72, 232) verbunden ist.

8. Verfahren nach Anspruch 4, wobei der Schritt des Einstellens einer Phase wenigstens einer der Mikrowellen das Einstellen von Phasen des ersten Paars von Mikrowellen umfasst.

9. Verfahren nach Anspruch 4, wobei der Schritt des Einstellens einer Phase wenigstens einer der Mikrowellen das Einstellen von Phasen des zweiten Paars von Mikrowellen umfasst.

10. Verfahren nach Anspruch 4, wobei der Schritt des Einstellens einer Phase wenigstens einer der Mikrowellen das Einstellen von Phasen sowohl des ersten Paars als auch des zweiten Paars von Mikrowellen umfasst.

11. Mikrowellen-Plasmadüsenanordnungseinheit, die umfasst:
einen Mikrowellenhohlraum (32, 92, 113, 113', 133, 142, 250, 276, 332); und
eine Anordnung von Düsen (37, 99, 337), die wenigstens teilweise in dem Mikrowellenhohlraum angeordnet sind, wobei jede der Düsen (36, 36', 98, 128, 144, 160, 170, 180, 190, 200, 212, 256, 256', 336) umfasst:
eine Gasströmungsröhre (120, 134, 148, 164, 174, 184, 194, 204, 216, 358), die geeignet ist, eine Gasströmung hindurch zu leiten, und die einen Einlassabschnitt und einen Auslassabschnitt hat, wobei der Auslassabschnitt außerhalb des Mikrowellenhohlraums (32, 92, 113, 113', 133, 142, 250, 276, 332) bereitgestellt ist; und
einen stangenförmigen Leiter (114, 130, 152, 162, 172, 182, 192, 202, 214, 352, 400, 408, 412, 416, 421, 424, 428, 434), der axial in der Gasströmungsröhre (120, 134, 148, 164, 174, 184, 194, 204, 216, 358) angeordnet ist, wobei der stangenförmige Leiter (114, 130, 152, 162, 172, 182, 192, 202, 214, 352, 400, 408, 412, 416, 421, 424, 428, 434) eine Spitze (117, 410, 414) und einen Sammelabschnitt (116, 354) hat, der in dem Mikrowellenhohlraum (32, 92, 113, 113', 133, 142, 250, 276, 332) angeordnet ist, um Mikrowellen an dem Sammelabschnitt (116, 354) zu empfangen, und der die empfangenen Mikrowellen sendet und
wobei die Spitze (117, 410, 414) benachbart zu dem Auslassabschnitt der Gasströmungsröhre (120, 134, 148, 164, 174, 184, 194, 204, 216, 358) positioniert ist.

12. Mikrowellen-Plasmadüsenanordnungseinheit nach Anspruch 11, wobei jede der Düsen (36, 36', 98, 128, 144, 160, 170, 180, 190, 200, 212, 256, 256', 336) ferner umfasst:
eine Wirbelführung (118, 132, 146, 166, 176, 186, 196, 206, 218), die zwischen dem stangenförmigen Leiter (114, 130, 152, 162, 172, 182, 192, 202, 214, 352, 400, 408, 412, 416, 421, 424, 428, 434) und der Gasströmungsröhre (120, 134, 148, 164, 174, 184, 194, 204, 216, 358) angeordnet ist, wobei die Wirbelführung (118, 132, 146, 166, 176, 186, 196, 206, 218) wenigstens einen Durchgang hat, um einem Gas, das entlang des wenigstens einen Durchgangs verläuft, eine spiralförmige Strömungsrichtung um den stangenförmigen Leiter (114, 130, 152, 162, 172, 182, 192, 202, 214, 352, 400, 408, 412, 416, 421, 424, 428, 434 zu verleihen.

13. Mikrowellen-Plasmadüsenanordnungseinheit nach Anspruch 12, wobei der Mikrowellenhohlraum (32, 92, 113, 113', 133, 142, 250, 276, 332) eine Wand (111, 143, 332) umfasst, wobei die Wand (111, 143, 332) des Mikrowellenhohlraums (32, 92, 113, 113', 133, 142, 250, 276, 332) einen Abschnitt eines Gasströmungsdurchgangs (112, 127) bildet, der betriebsfähig mit dem Einlassabschnitt der Gasströmungsröhre (120, 134, 148, 164, 174, 184, 194, 204, 216, 358) verbunden ist.

14. Mikrowellen-Plasmadüsenanordnungseinheit nach Anspruch 11, wobei jede der Düsen (36, 36', 98, 128, 144, 160, 170, 180, 190, 200, 212, 256, 256', 336) ferner umfasst:
eine Abschirmung (168, 210), die benachbart zu einem Abschnitt der Gasströmungsröhre (120, 134, 148, 164, 174, 184, 194, 204, 216, 358) angeordnet ist, um einen Mikrowellenleistungsverlust durch die Gasströmungsröhre (120, 134, 148, 164, 174, 184, 194, 204, 216, 358) zu verringern, wobei die Abschirmung (168, 210) aus einem leitenden Material gefertigt ist.

15. Mikrowellen-Plasmadüsenanordnungseinheit nach Anspruch 11, wobei jede der Düsen (36, 36', 98, 128, 144, 160, 170, 180, 190, 200, 212, 256, 256', 336) ferner umfasst:
eine geerdete Abschirmung (178, 360), die auf einer Außenoberfläche der Gasströmungsröhre (120, 134, 148, 164, 174, 184, 194, 204, 216, 358) angeordnet ist, um eine Mikrowellenverlustleistung durch die Gasströmungsröhre (120, 134, 148, 164, 174, 184, 194, 204, 216, 358) zu verringern, wobei die geerdete Abschirmung (178, 360) ein Loch zum Aufnehmen der Gasströmung hindurch hat.

16. Mikrowellen-Plasmadüsenanordnungseinheit nach Anspruch 15, wobei jede der Düsen (36, 36', 98, 128, 144, 160, 170, 180, 190, 200, 212, 256, 256', 336) ferner umfasst:
eine Positionshalterung (356), die zwischen dem stangenförmigen Leiter (114, 130, 152, 162, 172, 182, 192, 202, 214, 352, 400, 408, 412, 416, 421, 424, 428, 434) und der geerdeten Abschirmung (178, 360) angeordnet ist, um den stangenförmigen Leiter (114, 130, 152, 162, 172, 182, 192, 202, 214, 352, 400, 408, 412, 416, 421, 424, 428, 434) sicher relativ zu der geerdeten Abschirmung (178, 360) zu halten.

17. Mikrowellen-Plasmadüsenanordnungseinheit nach Anspruch 11, wobei die Gasströmungsröhre (120, 134, 148, 164, 174, 184, 194, 204, 216, 358) aus Quarz gefertigt ist.

18. Mikrowellen-Plasmadüsenanordnungseinheit nach Anspruch 11, wobei jede der Düsen (36, 36', 98, 128, 144, 160, 170, 180, 190, 200, 212, 256, 256', 336) ferner ein Paar Magnete (188, 198, 208) umfasst, die benachbart zu der Gasströmungsröhre (120, 134, 148, 164, 174, 184, 194, 204, 216, 358) angeordnet sind, wobei das Paar Magnete (188, 198, 208) eine Form hat, die einen Abschnitt eines Zylinders annähert.

19. Mikrowellen-Plasmadüsenanordnungseinheit nach Anspruch 11, wobei jede der Düsen (36, 36', 98, 128, 144, 160, 170, 180, 190, 200, 212, 256, 256', 336) ferner umfasst:
eine Anode (220), die benachbart zu einem Abschnitt der Gasströmungsröhre (120, 134, 148, 164, 174, 184, 194, 204, 216, 358) angeordnet ist; und
eine Kathode (222), die benachbart zu einem anderen Abschnitt der Gasströmungsröhre (120, 134, 148, 164, 174, 184, 194, 204, 216, 358) angeordnet ist.

20. Mikrowellen-Plasmadüsenanordnungseinheit nach Anspruch 11, wobei der Mikrowellenhohlraum (32, 92, 113, 113', 133, 142, 250, 276, 332) umfasst:
einen Mikrowelleneinlass; und
einen Kurzschlussschieber (94, 254a, 254b), der aufgebaut ist, um Mikrowellen zu reflektieren, die durch den Mikrowelleneinlass gesendet werden.

21. Mikrowellen-Plasmadüsenanordnungseinheit nach Anspruch 11, wobei der Mikrowellenhohlraum (32, 92, 113, 113', 133, 142, 250, 276, 332) umfasst:
zwei Mikrowelleneinlässe die in entgegengesetzten Seiten des Mikrowellenhohlraums (32, 92, 113, 113', 133, 142, 250, 276, 332) angeordnet sind.

22. Mikrowellen-Plasmadüsenanordnungseinheit nach Anspruch 11, wobei der Mikrowellenhohlraum (32, 92, 113, 113', 133, 142, 250, 276, 332) umfasst:
zwei Mikrowelleneinlässe, die in Seiten des Mikrowellenhohlraums (32, 92, 113, 113', 133, 142, 250, 276, 332) angeordnet sind, die normal zueinander sind; und
zwei Kurzschlussschieber (94, 254a, 254b), die aufgebaut sind, um von den Einlässen empfangene Mikrowellen zu reflektieren.

23. Mikrowellen-Plasmadüsenanordnungseinheit nach Anspruch 11, wobei der Mikrowellenhohlraum (32, 92, 113, 113', 133, 142, 250, 276, 332) umfasst:
ein erstes Paar von Mikrowelleneinlässen, die in entgegengesetzten Seiten des Mikrowellenhohlraums (32, 92, 113, 113', 133, 142, 250, 276, 332) entlang einer ersten Achse angeordnet sind;
ein zweites Paar von Mikrowelleneinlässen, die in entgegengesetzten Seiten des Mikrowellenhohlraum (32, 92, 113, 113', 133, 142, 250, 276, 332) entlang einer zweiten Achse angeordnet sind, wobei die zweite Achse im Wesentlichen normal zu der ersten Achse ist.

24. Mikrowellen-Plasmadüsenanordnungseinheit nach Anspruch 11, wobei der Mikrowellenhohlraum (32, 92, 113, 113', 133, 142, 250, 276, 332) aufgebaut ist, um unter Verwendung von in ihn geleiteten Mikrowellen mehrere stationäre Hochenergiebereiche (69, 69', 258, 262a, 262b, 268, 268') zu erzeugen, und wobei der Abschnitt des stangenförmigen Leiters (114, 130, 152, 162, 172, 182, 192, 202, 214, 352, 400, 408, 412, 416, 421, 424, 428, 434) innerhalb des Raums angeordnet ist, der durch die stationären Hochenergiebereiche (69, 69', 258, 262a, 262b, 268, 268') besetzt ist.

25. Mikrowellenplasmasystem, das umfasst:
eine Mikrowellen-Plasmadüsenanordnungseinheit nach einem der Ansprüche 11 bis 24;
einen Isolator (17a, 17b, 74, 237a, 237b), der betriebsfähig mit der Mikrowellenquelle verbunden ist;
einen Mikrowellenhohlraum (32, 92, 113, 113', 133, 142, 250, 276, 332) mit einem Einlass;
einen Wellenleiter (20a, 20b, 90, 272a-d), der betriebsfähig mit dem Isolator (17a, 17b, 74, 237a, 237b) und mit dem Einlass des Mikrowellenhohlraums (32, 92, 113, 113', 133, 142, 250, 276, 332) verbunden ist;
einen nicht rotierenden Phasenschieber (24a, 24b, 82, 244a, 244b), der betriebsfähig mit dem Wellenleiter (20a, 20b, 90, 272a-d) und dem Isolator (17a, 17b, 74, 237a, 237b) verbunden ist;
einen Zirkulator (16, 22a, 22b, 78, 80, 236a, 236b, 242a, 242b), der betriebsfähig mit dem Wellenleiter (20a, 20b, 90, 272a-d) verbunden ist und aufgebaut ist, um Mikrowellen zu dem nicht rotierenden Phasenschieber (24a, 24b, 82, 244a, 244b) zu leiten; und
einen Kurzschlussschieber (94, 254a, 254b), der betriebsfähig mit dem Mikrowellenhohlraum (32, 92, 113, 113', 133, 142, 250, 276, 332) verbunden ist.

26. Mikrowellenplasmasystem nach Anspruch 25, wobei jede der Düsen (36, 36', 98, 128, 144, 160, 170, 180, 190, 200, 212, 256, 256', 336) ferner umfasst:
eine Wirbelführung (118, 132, 146, 166, 176, 186, 196, 206, 218), die zwischen dem stangenförmigen Leiter (114, 130, 152, 162, 172, 182, 192, 202, 214, 352, 400, 408, 412, 416, 421, 424, 428, 434) und der Gasströmungsröhre (120, 134, 148, 164, 174, 184, 194, 204, 216, 358) angeordnet ist, wobei die Wirbelführung (118, 132, 146, 166, 176, 186, 196, 206, 218) wenigstens einen Durchgang hat, um einem Gas, das entlang des wenigstens einen Durchgangs verläuft, eine spiralförmige Strömungsrichtung um den stangenförmigen Leiter (114, 130, 152, 162, 172, 182, 192, 202, 214, 352, 400, 408, 412, 416, 421, 424, 428, 434 zu verleihen.

27. Mikrowellenplasmasystem nach Anspruch 26, wobei der Mikrowellenhohlraum (32, 92, 113, 113', 133, 142, 250, 276, 332) eine Wand (111, 143, 332) umfasst, wobei die Wand (111, 143, 332) des Mikrowellenhohlraums (32, 92, 113, 113', 133, 142, 250, 276, 332) einen Abschnitt eines Gasströmungsdurchgangs (112, 127) bildet, der betriebsfähig mit dem Einlassabschnitt der Gasströmungsröhre (120, 134, 148, 164, 174, 184, 194, 204, 216, 358) verbunden ist.

28. Mikrowellenplasma nach Anspruch 25, wobei jede der Düsen (36, 36', 98, 128, 144, 160, 170, 180, 190, 200, 212, 256, 256', 336) ferner umfasst:
eine Abschirmung (168, 210), die benachbart zu einem Abschnitt der Gasströmungsröhre (120, 134, 148, 164, 174, 184, 194, 204, 216, 358) angeordnet ist, um den Mikrowellenleistungsverlust durch die Gasströmungsröhre (120, 134, 148, 164, 174, 184, 194, 204, 216, 358) zu verringern, wobei die Abschirmung (168, 210) aus einem leitenden Material gefertigt ist.

29. Mikrowellenplasmasystem nach Anspruch 25, wobei jede der Düsen (36, 36', 98, 128, 144, 160, 170, 180, 190, 200, 212, 256, 256', 336) ferner umfasst:
eine geerdete Abschirmung (178, 360), die auf einer Außenfläche der Gasströmungsröhre (120, 134, 148, 164, 174, 184, 194, 204, 216, 358) angeordnet ist, um einen Mikrowellenleistungsverlust durch die Gasströmungsröhre (120, 134, 148, 164, 174, 184, 194, 204, 216, 358) zu verringern, wobei die geerdete Abschirmung (178, 360) ein Loch zum Aufnehmen der Gasströmung durch es hindurch hat.

30. Mikrowellenplasmasystem nach Anspruch 29, wobei jede der Düsen (36, 36', 98, 128, 144, 160, 170, 180, 190, 200, 212, 256, 256', 336) ferner umfasst:
eine Positionshalterung (356), die zwischen dem stangenförmigen Leiter (114, 130, 152, 162, 172, 182, 192, 202, 214, 352, 400, 408, 412, 416, 421, 424, 428, 434) und der geerdeten Abschirmung (178, 360) angeordnet ist, um den stangenförmigen Leiter (114, 130, 152, 162, 172, 182, 192, 202, 214, 352, 400, 408, 412, 416, 421, 424, 428, 434) relativ zu der geerdeten Abschirmung (178, 360) sicher zu halten.

31. Mikrowellenplasmasystem nach Anspruch 25, wobei die Gasströmungsröhre (120, 134, 148, 164, 174, 184, 194, 204, 216, 358) aus Quarz gefertigt ist.

32. Mikrowellenplasmasystem nach Anspruch 25, wobei jede der Düsen (36, 36', 98, 128, 144, 160, 170, 180, 190, 200, 212, 256, 256', 336) ferner ein Paar Magnete (188, 198, 208) umfasst, die benachbart zu der Gasströmungsröhre (120, 134, 148, 164, 174, 184, 194, 204, 216, 358) angeordnet sind, wobei das Paar Magnete (188, 198, 208) eine Form hat, die einen Abschnitt eines Zylinders annähert.

33. Mikrowellenplasmasystem nach Anspruch 25, wobei jede der Düsen (36, 36', 98, 128, 144, 160, 170, 180, 190, 200, 212, 256, 256', 336) ferner umfasst:
eine Anode (220), die benachbart zu einem Abschnitt der Gasströmungsröhre (120, 134, 148, 164, 174, 184, 194, 204, 216, 358) angeordnet ist; und
eine Kathode (222), die benachbart zu einem anderen Abschnitt der Gasströmungsröhre (120, 134, 148, 164, 174, 184, 194, 204, 216, 358) angeordnet ist.

34. Mikrowellenplasmasystem nach Anspruch 25, wobei der Mikrowellenhohlraum (32, 92, 113, 113', 133, 142, 250, 276, 332) aufgebaut ist, um unter Verwendung von in ihn geleiteten Mikrowellen mehrere stationäre Hochenergiebereiche (69, 69', 258, 262a, 262b, 268, 268') zu erzeugen, und wobei der Abschnitt (116, 354) des stangenförmigen Leiters (114, 130, 152, 162, 172, 182, 192, 202, 214, 352, 400, 408, 412, 416, 421, 424, 428, 434) innerhalb des Raums angeordnet ist, der durch die stationären Hochenergiebereiche (69, 69', 258, 262a, 262b, 268, 268') belegt ist.

35. Mikrowellenplasmasystem nach Anspruch 25, wobei der Isolator (17a, 17b, 74, 237a, 237b) umfasst:
einen Zirkulator (16, 22a, 22b, 78, 80, 236a, 236b, 242a, 242b), der betriebsfähig mit dem Wellenleiter (20a, 20b, 90, 272a-d) verbunden ist; und
eine Ersatzlast (18a, 18b, 76, 238a, 238b), die betriebsfähig mit dem Zirkulator (16, 22a, 22b, 78, 80, 236a, 242a, 242b) verbunden ist.

36. Mikrowellenplasmasystem nach Anspruch 25, das ferner umfasst:
eine Abstimmvorrichtung (30a, 30b, 88, 248a, 248b), die betriebsfähig mit dem Wellenleiter (20a, 20b, 90, 272a-d) und dem Mikrowellenhohlraum (32, 92, 113, 113', 133, 142, 250, 276, 332) verbunden ist.

37. Mikrowellenplasmasystem nach Anspruch 25, das ferner umfasst:
einen Koppler (26a, 26b, 86, 246a, 246b), der betriebsfähig mit dem Wellenleiter (20a, 20b, 90, 272a-d) und einem Leistungsmesser (28a, 28b, 84, 247a, 247b) zum Messen von Mikrowellenflüssen verbunden ist.

38. Mikrowellenplasmasystem nach einem der Ansprüche 25 bis 37, das umfasst:
ein Paar Isolatoren (17a, 17b, 74, 237a, 237b);
ein Paar Einlässe;
ein Paar Wellenleiter (20a, 20b, 90, 272a-d), wobei jeder der Wellenleiter (20a, 20b, 90, 272a-d) betriebsfähig mit einem entsprechenden der Isolatoren (17a, 17b, 74, 237a, 237b) und einem entsprechenden der Einlässe des Mikrowellenhohlraums (32, 92, 113, 113', 133, 142, 250, 276, 332) verbunden ist; und
ein Paar nicht rotierender Phasenschieber (24a, 24b, 82, 244a, 244b), wobei jeder der nicht rotierenden Phasenschieber (24a, 24b, 82, 244a, 244b) betriebsfähig mit einem entsprechenden der Wellenleiter (20a, 20b, 90, 272a-d) und mit einem entsprechenden der Isolatoren (17a, 17b, 74, 237a, 237b) verbunden ist.

39. Mikrowellenplasmasystem nach Anspruch 38, das ferner umfasst:
ein Paar Abstimmvorrichtungen (30a, 30b, 88, 248a, 248b), wobei jede der Abstimmvorrichtungen (30a, 30b, 88, 248a, 248b) betriebsfähig mit einem entsprechenden der Wellenleiter (20a, 20b, 90, 272a-d) und dem Mikrowellenhohlraum (32, 92, 113, 113', 133, 142, 250, 276, 332) verbunden ist.

40. Mikrowellenplasmasystem nach Anspruch 38, das ferner umfasst:
ein Paar Zirkulatoren (16, 22a, 22b, 78, 80, 236a, 236b, 242a, 242b), wobei jeder der Zirkulatoren (16, 22a, 22b, 78, 80, 236a, 236b, 242a, 242b) betriebsfähig mit einem entsprechenden der Wellenleiter (20a, 20b, 90, 272a-d) verbunden ist und aufgebaut ist, um Mikrowellen zu einem entsprechenden der nicht rotierenden Phasenschieber (24a, 24b, 82, 244a, 244b) zu leiten.

41. Mikrowellenplasmasystem nach Anspruch 38, das ferner umfasst:
ein Paar Koppler (26a, 26b, 86, 246a, 246b), wobei jeder der Koppler (26a, 26b, 86, 246a, 246b) betriebsfähig mit einem entsprechenden der Wellenleiter (20a, 20b, 90, 272a-d) und einem Leistungsmesser (28a, 28b, 84, 247a, 247b) zum Messen von Mikrowellenflüssen verbunden ist.

42. Mikrowellenplasmasystem nach Anspruch 38, wobei die Mikrowellenquelle (13, 233) ein Paar Mikrowellenleistungsköpfe (12, 72, 232) umfasst, wobei jeder der Mikrowellenleistungsköpfe (12, 72, 232) betriebsfähig mit einem entsprechenden der Isolatoren (17a, 17b, 74, 237a, 27b) verbunden ist.

43. Mikrowellenplasmasystem nach Anspruch 38, wobei die Mikrowellenquelle (13, 233) umfasst:
einen Mikrowellenleistungskopf (12, 72, 232) zum Erzeugen von Mikrowellen; und
einen Leistungsteiler (14, 234) zum Empfangen, Halbieren und Leiten der Mikrowellen zu den Isolatoren (17a, 17b, 74, 237a, 237b).

44. Mikrowellenplasmasystem nach Anspruch 38, das umfasst:
ein Paar Kurzschlussschieber (94, 254a, 254b).

45. Mikrowellenplasmasystem nach einem der Ansprüche 25 bis 37, das umfasst:
vier Einlässe;
vier Wellenleiter (20a, 20b, 90, 272a-d), wobei jeder der Wellenleiter (20a, 20b, 90, 272a-d) betriebsfähig mit einem entsprechenden der Einlässe des Mikrowellenhohlraums (32, 92, 113, 113', 133, 142, 250, 276, 332) und der Mikrowellenquelle (13, 233) verbunden ist;
vier nicht rotierende Phasenschieber (24a, 24b, 82, 244a, 244b), wobei jeder der nicht rotierenden Phasenschieber (24a, 24b, 82, 244a, 244b) betriebsfähig mit einem entsprechenden der Wellenleiter (20a, 20b, 90, 272a-d) und der Mikrowellenquelle (13, 233) verbunden sind;
vier Zirkulatoren (16, 22a, 22b, 78, 80, 236a, 236b, 242a, 242b), wobei jeder der Zirkulatoren (16, 22a, 22b, 78, 80, 236a, 236b, 242a, 242b) betriebsfähig mit einem entsprechenden der Wellenleiter (20a, 20b, 90, 272a-d) verbunden ist und aufgebaut ist, um von der Mikrowellenquelle (13, 233) erzeugte Mikrowellen zu wenigstens einem der nicht rotierenden Phasenschieber (24a, 24b, 82, 244a, 244b) zu leiten.

46. Mikrowellenplasmasystem nach Anspruch 45, wobei die Mikrowellenquelle (13, 233) umfasst:
vier Mikrowellenleistungsköpfe (12, 72, 232); und
vier Isolatoren (17a, 17b, 74, 237a, 237b), wobei jeder der Isolatoren (17a, 17b, 74, 237a, 237b) betriebsfähig mit einem entsprechenden der Mikrowellenleistungsköpfe (12, 72, 232) und wenigstens einem der Wellenleiter (20a, 20b, 90, 272a-d) verbunden ist, wobei jeder der Isolatoren (17a, 17b, 74, 237a, 237b) umfasst:
einen Zirkulator (16, 22a, 22b, 78, 80, 236a, 236b, 242a, 242b), der betriebsfähig mit dem Wellenleiter (20a, 20b, 90, 272a-d) verbunden ist; und
eine Ersatzlast (18a, 18b, 76, 238a, 238b), die betriebsfähig mit dem Zirkulator (16, 22a, 22b, 78, 80, 236a, 236b, 242a, 242b) verbunden ist.

47. Mikrowellenplasmasystem nach Anspruch 45, wobei die Mikrowellenquelle (13, 233) umfasst:
zwei Mikrowellenleistungsköpfe (12, 72, 232);
zwei Isolatoren (17a, 17b, 74, 237a, 237b), wobei jeder der Isolatoren (17a, 17b, 74, 237a, 237b) mit einem entsprechenden der Mikrowellenleistungsköpfe (12, 72, 232) verbunden ist, wobei jeder der Isolatoren (17a, 17b, 74, 237a, 237b) umfasst:
einen Zirkulator (16, 22a, 22b, 78, 80, 236a, 236b, 242a, 242b), der betriebsfähig mit dem Wellenleiter (20a, 20b, 90, 272a-d) verbunden ist; und
eine Ersatzlast (18a, 18b, 76, 238a, 238b), die betriebsfähig mit dem Zirkulator (16, 22a, 22b, 78, 80, 236a, 236b, 242a, 242b) verbunden ist; und
zwei Leistungsteiler (14, 234), wobei jeder der Leistungsteiler (14, 234) betriebsfähig mit einem entsprechenden der Isolatoren (17a, 17b, 74, 237a, 237b) verbunden ist, wobei jeder der Leistungsteiler (14, 234) aufgebaut ist, um die Mikrowellen zu empfangen, zu halbieren und zu entsprechenden zwei der Wellenleiter (20a, 20b, 90, 272a-d) zu leiten.

48. Mikrowellenplasma nach Anspruch 45, wobei die Mikrowellenquelle (13, 233) umfasst:
einen Mikrowellenleistungskopf (12, 72, 232);
einen Isolator (17a, 17b, 74, 237a, 237b), der betriebsfähig mit dem Mikrowellenleistungskopf (12, 72, 232) verbunden ist, wobei der Isolator (17a, 17b, 74, 237a, 237b) umfasst:
einen Zirkulator (16, 22a, 22b, 78, 80, 236a, 236b, 242a, 242b), der betriebsfähig mit dem Wellenleiter (20a, 20b, 90, 272a-d) verbunden ist; und
eine Ersatzlast (18a, 18b, 76, 238a, 238b), die betriebsfähig mit dem Zirkulator (16, 22a, 22b, 78, 80, 236a, 236b, 242a, 242b) verbunden ist; und
einen Leistungsteiler (14, 234), der mit dem Isolator (17a, 17b, 74, 237a, 237b) verbunden ist, wobei der Leistungsteiler (14, 234) aufgebaut ist, um die Mikrowellen zu empfangen, zu teilen und zu einem entsprechenden der Wellenleiter (20a, 20b, 90, 272a-d) zu leiten.

49. Mikrowellenplasmasystem nach Anspruch 45, das ferner umfasst:
vier Abstimmvorrichtrungen (30a, 30b, 88, 248a, 248b), wobei jede der Abstimmvorrichtungen (30a, 30b, 88, 248a, 248b) betriebsfähig mit einem entsprechenden der Wellenleiter (20a, 20b, 90, 272a-d) und dem Mikrowellenhohlraum (32, 92, 113, 113', 133, 142, 250, 276, 332) verbunden ist.

50. Mikrowellenplasmasystem nach Anspruch 45, das ferner umfasst:
vier Koppler (26a, 26b, 86, 246a, 246b), wobei jeder der Koppler (26a, 26b, 86, 246a, 246b) betriebsfähig mit einem entsprechenden der Wellenleiter (20a, 20b, 90, 272a-d) und einem Leistungsmesser (28a, 28b, 84, 247a, 247b) zum Messen von Mikrowellenflüssen verbunden ist.

## Revendications

1. Procédé de configuration d'un réseau de buses à plasma par micro-ondes, comprenant les étapes consistant à :
diriger des micro-ondes dans une cavité à micro-ondes (32, 92, 113, 113', 133, 142, 250, 276, 332) dans des directions opposées de sorte que les micro-ondes interfèrent et forment un motif de micro-ondes vertical qui est stationnaire au sein de la cavité à micro-ondes (32, 92, 113, 113', 133, 142, 250, 276, 332) ;
ajuster une phase d'au moins une des micro-ondes pour commander des régions à haute énergie (69, 69', 258, 262a, 262b, 269, 268') générées par le motif de micro-ondes vertical ; et
disposer un réseau de buses (37, 99, 337) au moins partiellement dans la cavité à micro-ondes (32, 92, 113, 113', 133, 142, 250, 276, 332) de sorte qu'un ou plusieurs éléments de buse (36, 36', 98, 128, 144, 160, 170, 180, 190, 200, 212, 256, 256', 336) du réseau de buses (37, 99, 337) sont configurés pour recevoir de l'énergie à micro-ondes provenant d'une région correspondante des régions à haute énergie (69, 69', 258, 262a, 262b, 269, 268'), dans lequel
chacune desdites buses (36, 36', 98,128, 144, 160, 170, 180, 190, 200, 212, 256, 256', 336) inclut un tube à écoulement gazeux (120, 134, 148, 164, 174, 184, 194, 204, 216, 358) adapté à diriger un écoulement gazeux à travers celui-ci et ayant une portion d'entrée et une portion de sortie,
ladite portion de sortie étant prévue en dehors de ladite cavité à micro-ondes (32, 92, 113, 113', 133, 142, 250, 276, 332), **caractérisé en ce que** chacune desdites buses inclut en outre
- un conducteur en forme de tige (114, 130, 152, 162, 172, 182, 192, 202, 214, 352, 400, 408, 412, 416, 421, 424, 428, 434) disposé de manière axiale dans ledit tube à écoulement gazeux (120, 134, 148, 164, 174, 184, 194, 204, 216, 358), ledit conducteur en forme de tige (114, 130, 152, 162, 172, 182, 192, 202, 214, 352, 400, 408, 412, 416, 421, 424, 428, 434) ayant une pointe (117, 410, 414) et une portion de collecte (116, 354) qui est disposée dans ladite cavité à micro-ondes (32, 92, 113, 113', 133, 142, 250, 276, 332) pour recevoir des micro-ondes au niveau de ladite portion de collecte (116, 354) et transmet les micro-ondes reçues le long de la surface de celle-ci et concentre les micro-ondes au niveau de ladite pointe (117, 410, 414) et dans lequel ladite pointe (117, 410, 414) est positionnée de manière adjacente à ladite portion de sortie dudit tube à écoulement gazeux (120, 134, 148, 164, 174, 184, 194, 204, 216, 358).

2. Procédé selon la revendication 1, dans lequel ladite étape consistant à diriger des micro-ondes inclut les étapes consistant à :
transmettre des micro-ondes vers la cavité à micro-ondes (32, 92, 113, 113', 133, 142, 250, 276, 332) ; et
refléter des micro-ondes en utilisant un court-circuit coulissant (94, 254a, 254b) connecté de manière opérationnelle à la cavité à micro-ondes (32, 92, 113, 113', 133, 142, 250, 276, 332).

3. Procédé selon la revendication 1, dans lequel ladite étape consistant à diriger des micro-ondes inclut l'étape consistant à :
transmettre des micro-ondes générées par deux têtes à énergie HF (12, 72, 232) vers la cavité à micro-ondes (32, 92, 113, 113', 133, 142, 250, 276, 332).

4. Procédé selon la revendication 1, comprenant les étapes consistant à :
diriger une seconde paire de micro-ondes dans la cavité à micro-ondes (32, 92, 113, 113', 133, 142, 250, 276, 332) dans des directions opposées le long d'un second axe, le premier axe étant perpendiculaire au second axe, de sorte que les première et seconde paires de micro-ondes interfèrent et forment des régions à haute énergie (69, 69', 258, 262a, 262b, 269, 268') qui sont stationnaires au sein de la cavité à micro-ondes (32, 92, 113, 113', 133, 142, 250, 276, 332).

5. Procédé selon la revendication 4, dans lequel ladite étape consistant à diriger la première paire de micro-ondes inclut les étapes consistant à :
transmettre des micro-ondes vers la cavité à micro-ondes (32, 92, 113, 113', 133, 142, 250, 276, 332) ; et
refléter des micro-ondes en utilisant un court-circuit coulissant (94, 254a, 254b) connecté de manière opérationnelle à la cavité à micro-ondes (32, 92, 113, 113', 133, 142, 250, 276, 332).

6. Procédé selon la revendication 4, dans lequel ladite étape consistant à diriger la première paire de micro-ondes inclut l'étape consistant à :
transmettre des micro-ondes générées par deux têtes à énergie HF (12, 72, 232) vers la cavité à micro-ondes (32, 92, 113, 113', 133, 142, 250, 276, 332).

7. Procédé selon la revendication 4, comprenant en outre les étapes consistant à :
générer les micro-ondes par une tête à énergie HF (12, 72, 232) ; et
fournir un diviseur de puissance (14, 234) connecté à la tête à énergie HF (12, 72, 232).

8. Procédé selon la revendication 4, dans lequel ladite étape consistant à ajuster une phase d'au moins une des micro-ondes inclut le fait d'ajuster des phases de la première paire de micro-ondes.

9. Procédé selon la revendication 4, dans lequel ladite étape consistant à ajuster une phase d'au moins une des micro-ondes inclut le fait d'ajuster des phases de la seconde paire de micro-ondes.

10. Procédé selon la revendication 4, dans lequel ladite étape consistant à ajuster une phase d'au moins une des micro-ondes inclut le fait d'ajuster des phases de la première paire et de la seconde paire de micro-ondes.

11. Module de réseau de buses à plasma par micro-ondes, comprenant :
une cavité à micro-ondes (32, 92, 113, 113', 133, 142, 250, 276, 332) ; et
un réseau de buses (37, 99, 337) disposées au moins partiellement dans la cavité à micro-ondes, chacune desdites buses (36, 36', 98, 128, 144, 160, 170, 180, 190, 200, 212, 256, 256', 336) incluant :
un tube à écoulement gazeux (120, 134, 148, 164, 174, 184, 194, 204, 216, 358) adapté à diriger un écoulement gazeux à travers celui-ci et ayant une portion d'entrée et une portion de sortie, ladite portion de sortie étant prévue en dehors de ladite cavité à micro-ondes (32, 92, 113, 113', 133, 142, 250, 276, 332) ; et
un conducteur en forme de tige (114, 130, 152, 162, 172, 182, 192, 202, 214, 352, 400, 408, 412, 416, 421, 424, 428, 434) disposé de manière axiale dans ledit tube à écoulement gazeux (120, 134, 148, 164, 174, 184, 194, 204, 216, 358), ledit conducteur en forme de tige (114, 130, 152, 162, 172, 182, 192, 202, 214, 352, 400, 408, 412, 416, 421, 424, 428, 434) ayant une pointe (117, 410, 414) et une portion de collecte (116, 354) qui est disposée dans ladite cavité à micro-ondes (32, 92, 113, 113', 133, 142, 250, 276, 332) pour recevoir des micro-ondes au niveau de ladite portion de collecte (116, 354) et transmet les micro-ondes reçues et
dans lequel ladite pointe (117, 410, 414) est positionnée de manière adjacente à ladite portion de sortie dudit tube à écoulement gazeux (120, 134, 148, 164, 174, 184, 194, 204, 216, 358).

12. Module de réseau de buses à plasma par micro-ondes selon la revendication 11, dans lequel chacune desdites buses (36, 36', 98, 128, 144, 160, 170, 180, 190, 200, 212, 256, 256', 336) inclut en outre :
un guide de vortex (118, 132, 146, 166, 176, 186, 196, 206, 218) disposé entre ledit conducteur en forme de tige (114, 130, 152, 162, 172, 182, 192, 202, 214, 352, 400, 408, 412, 416, 421, 424, 428, 434) et ledit tube à écoulement gazeux (120, 134, 148, 164, 174, 184, 194, 204, 216, 358), ledit guide de vortex (118, 132, 146, 166, 176, 186, 196, 206, 218) ayant au moins un passage pour conférer une direction d'écoulement de forme hélicoïdale autour dudit conducteur en forme de tige (114, 130, 152, 162, 172, 182, 192, 202, 214, 352, 400, 408, 412, 416, 421, 424, 428, 434) à un gaz passant le long dudit au moins un passage.

13. Module de réseau de buses à plasma par micro-ondes selon la revendication 12, dans lequel ladite cavité à micro-ondes (32, 92, 113, 113', 133, 142, 250, 276, 332) inclut une paroi (111, 143, 332), ladite paroi (111, 143, 332) de ladite cavité à micro-ondes (32, 92, 113, 113', 133, 142, 250, 276, 332) formant une portion d'un passage à écoulement gazeux (112, 127) connecté de manière opérationnelle à la portion d'entrée dudit tube à écoulement gazeux (120, 134, 148, 164, 174, 184, 194, 204, 216, 358).

14. Module de réseau de buses à plasma par micro-ondes selon la revendication 11, dans lequel chacune desdites buses (36, 36', 98, 128, 144, 160, 170, 180, 190, 200, 212, 256, 256', 336) inclut en outre :
un blindage (168, 210) disposé de manière adjacente à une portion dudit tube à écoulement gazeux (120, 134, 148, 164, 174, 184, 194, 204, 216, 358) pour réduire une perte d'énergie HF à travers ledit tube à écoulement gazeux (120, 134, 148, 164, 174, 184, 194, 204, 216, 358), ledit blindage (168, 210) étant réalisé en un matériau conducteur.

15. Module de réseau de buses à plasma par micro-ondes selon la revendication 11, dans lequel chacune desdites buses (36, 36', 98, 128, 144, 160, 170, 180, 190, 200, 212, 256, 256', 336) inclut en outre :
un blindage mis à la terre (178, 360) disposé sur une surface extérieure dudit tube à écoulement gazeux (120, 134, 148, 164, 174, 184, 194, 204, 216, 358) pour réduire une perte d'énergie HF à travers ledit tube à écoulement gazeux (120, 134, 148, 164, 174, 184, 194, 204, 216, 358), ledit blindage mis à la terre (178, 360) ayant un orifice pour recevoir l'écoulement gazeux à travers celui-ci.

16. Module de réseau de buses à plasma par micro-ondes selon la revendication 15, dans lequel chacune desdites buses (36, 36', 98, 128, 144, 160, 170, 180, 190, 200, 212, 256, 256', 336) inclut en outre :
un support de position (356) disposé entre ledit conducteur en forme de tige (114, 130, 152, 162, 172, 182, 192, 202, 214, 352, 400, 408, 412, 416, 421, 424, 428, 434) et ledit blindage mis à la terre (178, 360) pour maintenir de manière fixe ledit conducteur en forme de tige (114, 130, 152, 162, 172, 182, 192, 202, 214, 352, 400, 408, 412, 416, 421, 424, 428, 434) par rapport audit blindage mis à la terre (178, 360).

17. Module de réseau de buses à plasma par micro-ondes selon la revendication 11, dans lequel ledit tube à écoulement gazeux (120, 134, 148, 164, 174, 184, 194, 204, 216, 358) est réalisé en quartz.

18. Module de réseau de buses à plasma par micro-ondes selon la revendication 11, dans lequel chacune desdites buses (36, 36', 98, 128, 144, 160, 170, 180, 190, 200, 212, 256, 256', 336) inclut en outre une paire d'aimants (188, 198, 208) disposés de manière adjacente audit tube à écoulement gazeux (120, 134, 148, 164, 174, 184, 194, 204, 216, 358), ladite paire d'aimants (188, 198, 208) ayant une forme approximant une portion d'un cylindre.

19. Module de réseau de buses à plasma par micro-ondes selon la revendication 11, dans lequel chacune desdites buses (36, 36', 98, 128, 144, 160, 170, 180, 190, 200, 212, 256, 256', 336) inclut en outre :
une anode (220) disposée de manière adjacente à une portion dudit tube à écoulement gazeux (120, 134, 148, 164, 174, 184, 194, 204, 216, 358) ; et
une cathode (222) disposée de manière adjacente à une autre portion dudit tube à écoulement gazeux (120, 134, 148, 164, 174, 184, 194, 204, 216, 358).

20. Module de réseau de buses à plasma par micro-ondes selon la revendication 11, dans lequel ladite cavité à micro-ondes (32, 92, 113, 113', 133, 142, 250, 276, 332) inclut :
une entrée de micro-ondes ; et
un court-circuit coulissant (94, 254a, 254b) configuré pour refléter des micro-ondes transmises à travers ladite entrée de micro-ondes.

21. Module de réseau de buses à plasma par micro-ondes selon la revendication 11, dans lequel ladite cavité à micro-ondes (32, 92, 113, 113', 133, 142, 250, 276, 332) inclut :
deux entrées de micro-ondes disposées dans des côtés opposés de ladite cavité à micro-ondes (32, 92, 113, 113', 133, 142, 250, 276, 332).

22. Module de réseau de buses à plasma par micro-ondes selon la revendication 11, dans lequel ladite cavité à micro-ondes (32, 92, 113, 113', 133, 142, 250, 276, 332) inclut :
deux entrées de micro-ondes disposées dans des côtés de ladite cavité à micro-ondes (32, 92, 113, 113', 133, 142, 250, 276, 332) qui sont perpendiculaires l'un à l'autre ; et
deux courts-circuits coulissants (94, 254a, 254b) configurés pour refléter des micro-ondes reçues par lesdites entrées.

23. Module de réseau de buses à plasma par micro-ondes selon la revendication 11, dans lequel ladite cavité à micro-ondes (32, 92, 113, 113', 133, 142, 250, 276, 332) inclut :
une première paire d'entrées de micro-ondes disposées dans des côtés opposés de ladite cavité à micro-ondes (32, 92, 113, 113', 133, 142, 250, 276, 332) le long d'un premier axe ;
une seconde paire d'entrées de micro-ondes disposées dans des côtés opposés de ladite cavité à micro-ondes (32, 92, 113, 113', 133, 142, 250, 276, 332) le long d'un second axe, le second axe étant sensiblement perpendiculairement au premier axe.

24. Module de réseau de buses à plasma par micro-ondes selon la revendication 11, dans lequel ladite cavité à micro-ondes (32, 92, 113, 113', 133, 142, 250, 276, 332) est configurée pour générer une pluralité de régions à haute énergie stationnaires (69, 69', 258, 262a, 262b, 269, 268') en utilisant des micro-ondes dirigées vers celles-ci et dans lequel ladite portion dudit conducteur en forme de tige (114, 130, 152, 162, 172, 182, 192, 202, 214, 352, 400, 408, 412, 416, 421, 424, 428, 434) est disposée au sein de l'espace occupé par lesdites régions à haute énergie stationnaires (69, 69', 258, 262a, 262b, 269, 268').

25. Système à plasma par micro-ondes, comprenant :
un module de réseau de buses à plasma par micro-ondes selon l'une quelconque des revendications 11 à 24 ;
un affaiblisseur non réciproque (17a, 17b, 74, 237a, 237b) connecté de manière opérationnelle à ladite source de micro-ondes (13, 233) ;
une cavité à micro-ondes (32, 92, 113, 113', 133, 142, 250, 276, 332) ayant une entrée ;
un guide d'ondes (20a, 20b, 90, 272 a-d) connecté de manière opérationnelle audit affaiblisseur non réciproque (17a, 17b, 74, 237a, 237b) et à ladite entrée de ladite cavité à micro-ondes (32, 92, 113, 113', 133, 142, 250, 276, 332) ;
un déphaseur non rotatif (24a, 24b, 82, 244a, 244b) connecté de manière opérationnelle audit guide d'ondes (20a, 20b, 90, 272 a-d) et audit affaiblisseur non réciproque (17a, 17b, 74, 237a, 237b) ;
un circulateur (16, 22a, 22b, 78, 80, 236a, 236b, 242a, 242b) connecté de manière opérationnelle audit guide d'ondes (20a, 20b, 90, 272 a-d) et configuré pour diriger des micro-ondes vers ledit déphaseur non rotatif (24a, 24b, 82, 244a, 244b) ; et
un court-circuit coulissant (94, 254a, 254b) connecté de manière opérationnelle à ladite cavité à micro-ondes (32, 92, 113, 113', 133, 142, 250, 276, 332).

26. Système à plasma par micro-ondes selon la revendication 25, dans lequel chacune desdites buses (36, 36', 98, 128, 144, 160, 170, 180, 190, 200, 212, 256, 256', 336) inclut en outre :
un guide de vortex (118, 132, 146, 166, 176, 186, 196, 206, 218) disposé entre ledit conducteur en forme de tige (114, 130, 152, 162, 172, 182, 192, 202, 214, 352, 400, 408, 412, 416, 421, 424, 428, 434) et ledit tube à écoulement gazeux (120, 134, 148, 164, 174, 184, 194, 204, 216, 358), ledit guide de vortex (118, 132, 146, 166, 176, 186, 196, 206, 218) ayant au moins un passage pour conférer une direction d'écoulement de forme hélicoïdale autour dudit conducteur en forme de tige (114, 130, 152, 162, 172, 182, 192, 202, 214, 352, 400, 408, 412, 416, 421, 424, 428, 434) à un gaz passant le long dudit au moins un passage.

27. Système à plasma par micro-ondes selon la revendication 26, dans lequel ladite cavité à micro-ondes (32, 92, 113, 113', 133, 142, 250, 276, 332) inclut une paroi (111, 143, 332), ladite paroi (111, 143, 332) de ladite cavité à micro-ondes (32, 92, 113, 113', 133, 142, 250, 276, 332) formant une portion d'un passage à écoulement gazeux (112, 127) connecté de manière opérationnelle à la portion d'entrée dudit tube à écoulement gazeux (120, 134, 148, 164, 174, 184, 194, 204, 216, 358).

28. Système à plasma par micro-ondes selon la revendication 25, dans lequel chacune desdites buses (36, 36', 98, 128, 144, 160, 170, 180, 190, 200, 212, 256, 256', 336) inclut en outre :
un blindage (168, 210) disposé de manière adjacente à une portion dudit tube à écoulement gazeux (120, 134, 148, 164, 174, 184, 194, 204, 216, 358) pour réduire une perte d'énergie HF à travers ledit tube à écoulement gazeux (120, 134, 148, 164, 174, 184, 194, 204, 216, 358), ledit blindage (168, 210) étant réalisé en un matériau conducteur.

29. Système à plasma par micro-ondes selon la revendication 25, dans lequel chacune desdites buses (36, 36', 98, 128, 144, 160, 170, 180, 190, 200, 212, 256, 256', 336) inclut en outre :
un blindage mis à la terre (178, 360) disposé sur une surface extérieure dudit tube à écoulement gazeux (120, 134, 148, 164, 174, 184, 194, 204, 216, 358) pour réduire une perte d'énergie HF à travers ledit tube à écoulement gazeux (120, 134, 148, 164, 174, 184, 194, 204, 216, 358), ledit blindage mis à la terre (178, 360) ayant un orifice pour recevoir l'écoulement gazeux à travers celui-ci.

30. Système à plasma par micro-ondes selon la revendication 25, dans lequel chacune desdites buses (36, 36', 98, 128, 144, 160, 170, 180, 190, 200, 212, 256, 256', 336) inclut en outre :
un support de position (356) disposé entre ledit conducteur en forme de tige (114, 130, 152, 162, 172, 182, 192, 202, 214, 352, 400, 408, 412, 416, 421, 424, 428, 434) et ledit blindage mis à la terre (178, 360) pour maintenir de manière fixe ledit conducteur en forme de tige (114, 130, 152, 162, 172, 182, 192, 202, 214, 352, 400, 408, 412, 416, 421, 424, 428, 434) par rapport audit blindage mis à la terre (178, 360).

31. Système à plasma par micro-ondes selon la revendication 25, dans lequel ledit tube à écoulement gazeux (120, 134, 148, 164, 174, 184, 194, 204, 216, 358) est réalisé en quartz.

32. Système à plasma par micro-ondes selon la revendication 25, dans lequel chacune desdites buses (36, 36', 98, 128, 144, 160, 170, 180, 190, 200, 212, 256, 256', 336) inclut en outre une paire d'aimants (188, 198, 208) disposés de manière adjacente audit tube à écoulement gazeux (120, 134, 148, 164, 174, 184, 194, 204, 216, 358), ladite paire d'aimants (188, 198, 208) ayant une forme approximant une portion d'un cylindre.

33. Système à plasma par micro-ondes selon la revendication 25, dans lequel chacune desdites buses (36, 36', 98, 128, 144, 160, 170, 180, 190, 200, 212, 256, 256', 336) inclut en outre :
une anode (220) disposée de manière adjacente à une portion dudit tube à écoulement gazeux (120, 134, 148, 164, 174, 184, 194, 204, 216, 358) ; et
une cathode (222) disposée de manière adjacente à une autre portion dudit tube à écoulement gazeux (120, 134, 148, 164, 174, 184, 194, 204, 216, 358).

34. Système à plasma par micro-ondes selon la revendication 25, dans lequel ladite cavité à micro-ondes (32, 92, 113, 113', 133, 142, 250, 276, 332) est configurée pour générer une pluralité de régions à haute énergie stationnaires (69, 69', 258, 262a, 262b, 269, 268') en utilisant des micro-ondes dirigées vers celles-ci et dans lequel ladite portion dudit conducteur en forme de tige (114, 130, 152, 162, 172, 182, 192, 202, 214, 352, 400, 408, 412, 416, 421, 424, 428, 434) est disposée au sein de l'espace occupé par lesdites régions à haute énergie stationnaires (69, 69', 258, 262a, 262b, 269, 268').

35. Système à plasma par micro-ondes selon la revendication 25, dans lequel ledit affaiblisseur non réciproque (17a, 17b, 74, 237a, 237b) inclut :
un circulateur (16, 22a, 22b, 78, 80, 236a, 236b, 242a, 242b) connecté de manière opérationnelle audit guide d'ondes (20a, 20b, 90, 272a-d) ; et
une charge fictive (18a, 18b, 76, 238a, 238b) connectée de manière opérationnelle audit circulateur (16, 22a, 22b, 78, 80, 236a, 236b, 242a, 242b).

36. Système à plasma par micro-ondes selon la revendication 25, comprenant en outre :
un syntoniseur (30a, 30b, 88, 248a, 248b) connecté de manière opérationnelle audit guide d'ondes (20a, 20b, 90, 272a-d) et à ladite cavité à micro-ondes (32, 92, 113, 113', 133, 142, 250, 276, 332).

37. Système à plasma par micro-ondes selon la revendication 25, comprenant en outre :
un coupleur (26a, 26b, 86, 246a, 246b) connecté de manière opérationnelle audit guide d'ondes (20a, 20b, 90, 272a-d) et un mesureur de puissance (28a, 28b, 84, 247a, 247b) pour mesurer des flux de micro-ondes.

38. Système à plasma par micro-ondes selon l'une quelconque des revendications 25 à 37, comprenant en outre :
une paire d'affaiblisseurs non réciproques (17a, 17b, 74, 237a, 237b);
une paire d'entrées ;
une paire de guides d'ondes (20a, 20b, 90, 272a-d), chacun desdits guides d'onde (20a, 20b, 90, 272a-d) étant connecté de manière opérationnelle à un affaiblisseur non réciproque correspondant desdits affaiblisseurs non réciproques (17a, 17b, 74, 237a, 237b) et à une entrée correspondante desdites entrées de ladite cavité à micro-ondes (32, 92, 113, 113', 133, 142, 250, 276, 332) ; et
une paire de déphaseurs non rotatifs (24a, 24b, 82, 244a, 244b), chacun desdits déphaseurs non rotatifs (24a, 24b, 82, 244a, 244b) étant connecté de manière opérationnelle à un guide d'ondes correspondant desdits guides d'ondes (20a, 20b, 90, 272a-d) et à un affaiblisseur non réciproque correspondant desdits affaiblisseurs non réciproques (17a, 17b, 74, 237a, 237b).

39. Système à plasma par micro-ondes selon la revendication 38, comprenant en outre :
une paire de syntoniseurs (30a, 30b, 88, 248a, 248b), chacun desdits syntoniseurs (30a, 30b, 88, 248a, 248b) étant connecté de manière opérationnelle à un guide d'ondes correspondant desdits guides d'ondes (20a, 20b, 90, 272a-d) et à ladite cavité à micro-ondes (32, 92, 113, 113', 133, 142, 250, 276, 332).

40. Système à plasma par micro-ondes selon la revendication 38, comprenant en outre :
une paire de circulateurs (16, 22a, 22b, 78, 80, 236a, 236b, 242a, 242b), chacun desdits circulateurs (16, 22a, 22b, 78, 80, 236a, 236b, 242a, 242b) étant connecté de manière opérationnelle à un guide d'ondes correspondant desdits guide d'ondes (20a, 20b, 90, 272 a-d) et configuré pour diriger des micro-ondes vers un déphaseur non rotatif correspondant desdits déphaseurs non rotatifs (24a, 24b, 82, 244a, 244b).

41. Système à plasma par micro-ondes selon la revendication 38, comprenant en outre :
une paire de coupleurs (26a, 26b, 86, 246a, 246b), chacun desdits coupleurs (26a, 26b, 86, 246a, 246b) étant connecté de manière opérationnelle à un guide d'ondes correspondant desdits guide d'ondes (20a, 20b, 90, 272a-d) et un mesureur de puissance (28a, 28b, 84, 247a, 247b) pour mesurer des flux de micro-ondes.

42. Système à plasma par micro-ondes selon la revendication 38, dans lequel ladite source de micro-ondes (13, 233) inclut une paire de têtes à énergie HF (12, 72, 232), chacune desdites têtes à énergie HF (12, 72, 232) étant connectée de manière opérationnelle à un affaiblisseur non réciproque correspondant desdits affaiblisseurs non réciproques (17a, 17b, 74, 237a, 237b).

43. Système à plasma par micro-ondes selon la revendication 38, dans lequel ladite source de micro-ondes (13, 233) inclut :
une tête à énergie HF (12, 72, 232) pour générer des micro-ondes ; et
un diviseur de puissance (14, 234) pour recevoir, bissecter et diriger les micro-ondes vers lesdits affaiblisseurs non réciproques (17a, 17b, 74, 237a, 237b).

44. Système à plasma par micro-ondes selon la revendication 38, comprenant :
une paire de courts-circuits coulissants (94, 254a, 254b).

45. Système à plasma par micro-ondes selon l'une quelconque des revendications 25 à 37, comprenant :
quatre entrées ;
quatre guides d'ondes (20a, 20b, 90, 272a-d), chacun desdits guides d'ondes (20a, 20b, 90, 272a-d) étant connecté de manière opérationnelle à une entrée correspondante desdites entrées de ladite cavité à micro-ondes (32, 92, 113, 113', 133, 142, 250, 276, 332) et à ladite source de micro-ondes (13, 233) ;
quatre déphaseurs non rotatifs (24a, 24b, 82, 244a, 244b), chacun desdits déphaseurs non rotatifs (24a, 24b, 82, 244a, 244b) étant connecté de manière opérationnelle à un guide d'ondes correspondant desdits guides d'ondes (20a, 20b, 90, 272a-d) et à ladite source de micro-ondes (13, 233) ;
quatre circulateurs (16, 22a, 22b, 78, 80, 236a, 236b, 242a, 242b), chacun desdits circulateurs (16, 22a, 22b, 78, 80, 236a, 236b, 242a, 242b) étant connecté de manière opérationnelle à un guide d'ondes correspondant desdits guides d'ondes (20a, 20b, 90, 272 a-d) et configuré pour diriger des micro-ondes générées par ladite source de micro-ondes (13, 233) vers au moins un desdits déphaseurs non rotatifs (24a, 24b, 82, 244a, 244b).

46. Système à plasma par micro-ondes selon la revendication 45, dans lequel ladite source de micro-ondes (13, 233) inclut :
quatre têtes à énergie HF (12, 72, 232) ; et
quatre affaiblisseurs non réciproques (17a, 17b, 74, 237a, 237b), chacun desdits affaiblisseurs non réciproques (17a, 17b, 74, 237a, 237b) étant connecté de manière opérationnelle à une tête à énergie HF correspondante desdites têtes à énergie HF (12, 72, 232) et à au moins un desdits guides d'ondes (20a, 20b, 90, 272 a-d), chacun desdits affaiblisseurs non réciproques (17a, 17b, 74, 237a, 237b) incluant :
un circulateur (16, 22a, 22b, 78, 80, 236a, 236b, 242a, 242b) connecté de manière opérationnelle audit guide d'ondes (20a, 20b, 90, 272a-d) ; et
une charge fictive (18a, 18b, 76, 238a, 238b) connectée de manière opérationnelle audit circulateur (16, 22a, 22b, 78, 80, 236a, 236b, 242a, 242b).

47. Système à plasma par micro-ondes selon la revendication 45, dans lequel ladite source de micro-ondes (13, 233) inclut :
deux têtes à énergie HF (12, 72, 232) ;
deux affaiblisseurs non réciproques (17a, 17b, 74, 237a, 237b), chacun desdits affaiblisseurs non réciproques (17a, 17b, 74, 237a, 237b) étant connecté à une tête à énergie HF correspondante desdites têtes à énergie HF (12, 72, 232),
chacun desdits affaiblisseurs non réciproques (17a, 17b, 74, 237a, 237b) incluant :
un circulateur (16, 22a, 22b, 78, 80, 236a, 236b, 242a, 242b) connecté de manière opérationnelle audit guide d'ondes (20a, 20b, 90, 272a-d) ; et
une charge fictive (18a, 18b, 76, 238a, 238b) connectée de manière opérationnelle audit circulateur (16, 22a, 22b, 78, 80, 236a, 236b, 242a, 242b); et
deux diviseurs de puissance (14, 234), chacun desdits diviseurs de puissance (14, 234) étant connecté de manière opérationnelle à un affaiblisseur non réciproque correspondant desdits affaiblisseurs non réciproques (17a, 17b, 74, 237a, 237b), chacun desdits diviseurs de puissance (14, 234) étant configuré pour recevoir, bissecter et diriger les micro-ondes vers deux guides d'ondes correspondants desdits guides d'ondes (20a, 20b, 90, 272a-d).

48. Système à plasma par micro-ondes selon la revendication 45, dans lequel ladite source de micro-ondes (13, 233) inclut :
une tête à énergie HF (12, 72, 232) ;
un affaiblisseur non réciproque (17a, 17b, 74, 237a, 237b) connecté de manière opérationnelle à ladite tête à énergie HF (12, 72, 232), ledit affaiblisseur non réciproque (17a, 17b, 74, 237a, 237b) incluant :
un circulateur (16, 22a, 22b, 78, 80, 236a, 236b, 242a, 242b) connecté de manière opérationnelle audit guide d'ondes (20a, 20b, 90, 272a-d) ; et
une charge fictive (18a, 18b, 76, 238a, 238b) connectée de manière opérationnelle audit circulateur (16, 22a, 22b, 78, 80, 236a, 236b, 242a, 242b); et
un diviseur de puissance (14, 234) connecté audit affaiblisseur non réciproque (17a, 17b, 74, 237a, 237b), ledit diviseur de puissance (14, 234) étant configuré pour recevoir, bissecter et diriger les micro-ondes vers un guide d'ondes correspondant desdits guides d'ondes (20a, 20b, 90, 272a-d).

49. Système à plasma par micro-ondes selon la revendication 45, comprenant en outre :
quatre syntoniseurs (30a, 30b, 88, 248a, 248b), chacun desdits syntoniseurs (30a, 30b, 88, 248a, 248b) étant connecté de manière opérationnelle à un guide d'ondes correspondant desdits guides d'ondes (20a, 20b, 90, 272a-d) et à ladite cavité à micro-ondes (32, 92, 113, 113', 133, 142, 250, 276, 332).

50. Système à plasma par micro-ondes selon la revendication 45, comprenant en outre :
quatre coupleurs (26a, 26b, 86, 246a, 246b), chacun desdits coupleurs (26a, 26b, 86, 246a, 246b) étant connecté de manière opérationnelle à un guide d'ondes correspondant desdits guide d'ondes (20a, 20b, 90, 272a-d) et un mesureur de puissance (28a, 28b, 84, 247a, 247b) pour mesurer des flux de micro-ondes.
